(19) Europäisches Patentamt
European Patent Office
Office européen des brevets

(11) **EP 4 143 751 B1**

(12) **EUROPEAN PATENT SPECIFICATION**

(45) Date of publication and mention
of the grant of the patent:
**01.07.2026 Bulletin 2026/27**

(21) Application number: **21796189.5**

(22) Date of filing: **27.04.2021**

(51) International Patent Classification (IPC):
**G16H 20/70** (2018.01)   **G16H 70/20** (2018.01)
**G16H 50/20** (2018.01)   **G06N 5/02** (2023.01)
**A61B 5/145** (2006.01)

(52) Cooperative Patent Classification (CPC):
**A61B 5/14532; G16H 20/70; G16H 50/20;
G16H 70/20;** Y02A 90/10

(86) International application number:
**PCT/US2021/029440**

(87) International publication number:
**WO 2021/222282 (04.11.2021 Gazette 2021/44)**

(54) **ADAPTIVE DECISION SUPPORT SYSTEMS**

ADAPTIVE ENTSCHEIDUNGSUNTERSTÜTZUNGSSYSTEME

SYSTÈMES D'AIDE À LA DÉCISION ADAPTATIFS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2020 US 202063016784 P**

(43) Date of publication of application:
**08.03.2023 Bulletin 2023/10**

(73) Proprietor: **Dexcom, Inc.
San Diego, CA 92121 (US)**

(72) Inventors:
• **SIMPSON, Peter C.
San Diego, California 92121 (US)**
• **CRAWFORD, Margaret Anne
San Diego, California 92121 (US)**
• **JOHNSON, Matthew Lawrence
San Diego, California 92121 (US)**
• **VYAS, Neha
San Diego, California 92121 (US)**
• **KAMATH, Apurv Ullas
San Diego, California 92121 (US)**

(74) Representative: **Crowell & Moring U.K. LLP
199 Bishopsgate
London EC2M 3TY (GB)**

(56) References cited:
US-A1- 2015 120 317   US-A1- 2017 329 917
US-A1- 2018 277 246   US-A1- 2018 277 246
US-A1- 2019 096 281   US-A1- 2019 252 079

**Description**

RELATED APPLICATIONS

This application claims benefit of U.S. Provisional Patent Application No. 63/016784, filed April 28, 2020.

BACKGROUND

Field

This application relates generally to medical devices such as analyte sensors, including systems and methods for using the same to provide decision-support guidance to a patient, a caregiver, a healthcare provider, or another user in order to help with improving the patient's health.

Description of the Related Technology

Diabetes is a metabolic condition relating to the production or use of insulin by the body. Insulin is a hormone that allows the body to use glucose for energy, or store glucose as fat.

When a person eats a meal that contains carbohydrates, the food is processed by the digestive system, which produces glucose in the person's blood. Blood glucose can be used for energy or stored as fat The body normally maintains blood glucose levels in a range that provides sufficient energy to support bodily functions and avoids problems that can arise when glucose levels are too high, or too lo w. Regulation of blood glucose levels depends on the production and use of insulin, which regulates the movement of blood glucose into cells.

When the body does not produce enough insulin, or when the body is unable to effectively use insulin that is present, blood sugar levels can elevate beyond normal ranges. The state of having a higher than normal blood sugar level is called "hyperglycemia." Chronic hyperglycemia can lead to a number of health problems, such as cardiovascular disease, cataract and other eye problems, nerve damage (neuropathy), and kidney damage. Hyperglycemia can also lead to acute problems, such as diabetic ketoacidosis-a state in which the body becomes excessively acidic due to the presence of blood glucose and ketones, which are produced when the body cannot use glucose. The state of having lower than normal blood glucose levels is called "hypoglycemia." Severe hypoglycemia can lead to acute crises that can result in seizures or death.

A diabetes patient can receive insulin to manage blood glucose levels. Insulin can be received, for example, through a manual injection with a needle. Wearable insulin pumps are also available. Diet and exercise also affect blood glucose levels.

Diabetes conditions are sometimes referred to as "Type 1" and "Type 2." A Type 1 diabetes patient is typically able to use insulin when it is present, but the body is unable to produce sufficient amounts of insulin, because of a problem with the insulin-producing beta cells of the pancreas. A Type 2 diabetes patient may produce some insulin, but the patient has become "insulin resistant" due to a reduced sensitivity to insulin. The result is that even though insulin is present in the body, the insulin is not sufficiently used by the patient's body to effectively regulate blood sugar levels.

Management of diabetes can present complex challenges for patients, clinicians, and caregivers, as a confluence of many factors can impact a patient's glucose level and glucose trends. To assist patients with better managing this condition, a variety of diabetes intervention software applications (hereinafter "applications") have been developed by various providers. However, these applications, which generally execute on patients' mobile devices, suffer from very high attrition rates in the early days, weeks, or months of use. Such high attrition rates are not just limited to diabetes intervention applications but are also generally applicable to most health-related applications. Such health-related applications may include intervention applications for supporting the treatment of various diseases as well as applications that help with improving a patient's health overall, such as weight loss applications.

This background is provided to introduce a brief context for the summary and detailed description that follow. This background is not intended to be an aid in determining the scope of the claimed subject matter nor be viewed as limiting the claimed subject matter to implementations that solve any or all of the disadvantages or problems presented above.

US2018277246 discloses infusion devices and related medical devices, patient data management systems, and methods for monitoring a physiological condition of a patient.

SUMMARY

The invention is as defined in the appended claims. One aspect is a system comprising: a memory circuit; and a processor configured to: receive a request to configure an application for use by a user, wherein the application is at least partially resident on a computing device to manage sensor data generated by a glucose monitoring system associated with the user; identify an objective for the user; identify classifying information associated with the user, the classifying information including at least one of the objective, interest, ability', demographic information, disease progression information, or medication regimen information of the user; select a group of users from among a pool of users based on one or more similarities between the user and the group of users with respect to the identified classifying information; identify one or more application features from a plurality of application features based on the objective of the user and a correlation of each of the plurality of application features with the objective in a dataset associated with the group of users; and automatically configure the application with the one or more application features.

In the above system, the processor being configured to identify the objective comprises the processor being configured to. receive user input relating to what the user intends to achieve with respect to the user's diabetes, and convert the user input into the objective based on one or more defined guidelines. In the above system, the processor being configured to convert the user input into the objective based on the one or more defined guidelines comprises the processor being configured to; categorize the user into a category' based on the guidelines and information associated with the user, wherein: the information associated with the user includes the classifying information, and the guidelines indicate the objective for the category; and select the objective for the user based on the categorization.

In the above system, the processor being configured to identify the objective comprises the processor being configured to: receive user input relating to what the user intends to achieve with respect to the user's diabetes; and convert the user input into the objective based on information associated with the group of users. In the above system, the information associated with the group of users includes one or more glucose-related metrics of the group of users. In the above system, the correlation of each of the plurality of application features with the objective comprises a correlation of each of the plurality of application features with achievement of the objective. In the above system, selecting the group of users is further based on a programmatic outcome metric of each of the pool of users with respect to the objective. In the above system, programmatic outcome metrics of the selected group of users are above a threshold programmatic outcome metric associated with achievement of the objective, and the threshold programmatic outcome metric associated with achievement of the objective is indicative of a defined minimum amount of positive progression towards achieving the objective.

In the above system, the correlation of each of the plurality of application features with the objective is based on a number of users in the selected group of users who used the feature and behavioral engagement of the number of users with respect to the feature. In the above system, behavioral engagement of each of the number of users with respect to the application feature is indicated by a behavioral engagement metric (BEM) of each user of the number of users with respect to the application feature, wherein the BEM is based on an interaction of each user of the number of users with the feature, the interaction including at least one of: a frequency with which each user of the number of users interacts with the application feature; a frequency with which each user of the number of users ignores a guidance generated by the application feature; an average amount of time each user of the number of users spends interacting with the application feature; or how closely behavior of each user of the number of users adheres to the guidance generated by the application feature. In the above system, each of the one or more application features has a correlation with the objective that is above a correlation threshold.

In the above system, the processor is further configured to: receive a plurality of inputs including: a first input including glucose measurements associated with the user generated by the glucose monitoring system; and a second input indicative of behavior of the user with respect to the one or more application features; calculate a programmatic outcome metric associated with the objective based at least on the first input, wherein the programmatic outcome metric is indicative of an extent to which the user has achieved the objective; calculate, based on the second input, one or more behavioral engagement metrics (BEMs) for the one or more application features, such that a separate BEM is calculated for each of the one or more application features; identify one or more users in the selected group of users or the pool of users with BEMs similar to the calculated one or more BEMs; identify a new application feature not included in the one or more features based on the feature being associated with a BEM above a threshold for at least one of the one or more users; and reconfigure the application with the new application feature based on at least one of the one or more BEMs and the programmatic outcome metric.

In the above system, each BEM of the one or more BEMs is based on an interaction of the user with a corresponding application feature of the one or more application features, the interaction including at least one of: a frequency with which the user interacts with the corresponding application feature; a frequency with which the user ignores a guidance generated by the corresponding application feature; an average amount of time the user spends interacting with the corresponding application feature; or how closely behavior of the user adheres to the guidance generated by the corresponding application feature. In the above system, the processor being configured to reconfigure the application with the new application feature comprises the processor being configured to: identify a low performing application feature of the one or more application features with a corresponding BEM that is below a threshold; and replace the low performing application feature of the one or more application features with the new application feature.

In the above system, the processor being configured to reconfigure the application with the new application feature comprises the processor being configured to: identify that the low performing application feature of the one or more application features relates to the objective; and identify that the programmatic outcome metric is below a threshold. In the above system, each application feature of the one or more application features comprises a feature setting.

Another aspect is a method of configuring an application with one or more application features, comprising: receiving a request to configure the application for use by a user, wherein the application is at least partially resident on a computing device to manage sensor data generated by a glucose monitoring system associated with the user; identifying an objective for the user; identifying classifying information associated with the user, the classifying information including at least one of the objective, interest, ability, demographic information, disease progression information, or medication regimen information of the user; selecting a group of users from among a pool of users based on one or more similarities between the user and the group of users with respect to the identified classifying information; identifying the one or more application features from a plurality of application features based on the objective of the user and a correlation of each of the plurality of application features with the objective in a dataset associated with the group of users; and configuring the application with the one or more application features.

In the above method, identifying the objective further comprises: receiving user input relating to what the user intends to achieve with respect to the user's diabetes; and converting the user input into the objective based on one or more defined guidelines. In the above method, the converting further comprises: categorizing the user into a category based on the guidelines and information associated with the user, wherein: the information associated with the user includes the classifying information, and the guidelines indicate the objective for the category; and selecting the objective for the user based on the categorization. In the above method, identifying the objective comprises: receiving user input relating to what the user intends to achieve with respect to the user's diabetes; and converting the user input into the objective based on information associated with the group of users.

In the above method, the information associated with the group of users includes one or more glucose-related metrics of the group of users. In the above method, the correlation of each of the plurality of application features with the objective comprises a correlation of each of the plurality of application features with achievement of the objective. In the above method, selecting the group of users is further based on a programmatic outcome metric of each of the pool of users with respect to the objective. In the above method, programmatic outcome metrics of the selected group of users are above a threshold programmatic outcome metric associated with achievement of the objective, and the threshold programmatic outcome metric associated with achievement of the objective is indicative of a defined minimum amount of positive progression towards achieving the objective.

In the above method, the correlation of each of the plurality of application features with the objective is based on a number of users in the selected group of users who used the feature and behavioral engagement of the number of users with respect to the feature. In the above method, behavioral engagement of each of the number of users with respect to the application feature is indicated by a behavioral engagement metric (BEM) of each user of the number of users with respect to the application feature, and wherein the BEM is based on an interaction of each user of the number of users with the feature, the interaction including at least one of: a frequency with which each user of the number of users interacts with the application feature; a frequency with which each user of the number of users ignores a guidance generated by the application feature; an average amount of time each user of the number of users spends interacting with the application feature; or how closely behavior of each user of the number of users adheres to the guidance generated by the application feature. In the above method, each of the one or more application features has a correlation with the objective that is above a correlation threshold.

The above method further comprises: receiving a plurality of inputs including: a first input including glucose measurements associated with the user generated by the glucose monitoring system; and a second input indicative of behavior of the user with respect to the one or more application features; calculating a programmatic outcome metric associated with the objective based at least on the first input, wherein the programmatic outcome metric is indicative of an extent to which the user has achieved the objective; calculating, based on the second input, one or more behavioral engagement metrics (BEMs) for the one or more application features, such that a separate BEM is calculated for each of the one or more application features; identifying one or more users in the selected group of users or the pool of users with BEMs similar to the calculated one or more BEMs; identifying a new application feature not included in the one or more features based on the feature being associated with a BEM above a threshold for at least one of the one or more users; and reconfiguring the application with the new application feature based on at least one of the one or more BEMs and the programmatic outcome metric.

In the above method, each BEM of the one or more BEMs is based on an interaction of the user with a corresponding application feature of the one or more application features, the interaction including at least one of: a frequency with which the user interacts with the corresponding application feature; a frequency with which the user ignores a guidance generated by the corresponding application feature; an average amount of time the user spends interacting with the corresponding application feature; or how closely behavior of the user adheres to the guidance generated by the corresponding application feature. In the above method, reconfiguring the application with the new application feature comprises: identifying a low performing application feature of the one or more application features with a corresponding BEM that is below a threshold; and replacing the low performing application feature of the one or more application features with the new application feature.

In the above method, reconfiguring the application with the new application feature comprises: identifying that the low performing application feature of the one or more application features relates to the objective; and identifying that the programmatic outcome metric is below a threshold. In the above method, each application feature of the one or more application features comprises a feature setting.

Another aspect is a non-transitory computer readable medium having instructions stored thereon that, when executed by a processor, causes a computing system to perform a method of configuring an application with one or more application features, the method comprising: receiving a request to configure the application for use by a user, wherein the application is at least partially resident on a computing device to manage sensor data generated by a glucose monitoring system associated with the user; identifying an objective for the user; identifying classifying information associated with the user, the classifying information including at least one of the objective, interest, ability, demographic information, disease progression information, or medication regimen information of the user; selecting a group of users from among a pool of users based on one or more similarities between the user and the group of users with respect to the identified classifying information; identifying the one or more application features from a plurality of application features based on the objective of the user and a correlation of each of the plurality of application features with the objective in a dataset associated with the group of users; and configuring the application with the one or more application features.

In the above medium, identifying the objective further comprises: receiving user input relating to what the user intends to achieve with respect to the user's diabetes; and converting the user input into the objective based on one or more defined guidelines. In the above medium, the converting further comprises: categorizing the user into a category based on the guidelines and information associated with the user, wherein: the information associated with the user includes the classifying information, and the guidelines indicate the objective for the category; and selecting the objective for the user based on the categorization. In the above medium, identifying the objective further comprises: receiving user input relating to what the user intends to achieve with respect to the user's diabetes; and converting the user input into the objective based on information associated with the group of users. In the above medium, the information associated with the group of users includes one or more glucose-related metrics of the group of users.

In the above medium, the correlation of each of the plurality of application features with the objective comprises a correlation of each of the plurality of application features with achievement of the objective. In the above medium, selecting the group of users is further based on a programmatic outcome metric of each of the pool of users with respect to the objective. In the above medium, programmatic outcome metrics of the selected group of users are above a threshold programmatic outcome metric associated with achievement of the objective, and the threshold programmatic outcome metric associated with achievement of the objective is indicative of a defined minimum amount of positive progression towards achieving the objective.

In the above medium, the correlation of each of the plurality of application features with the objective is based on a number of users in the selected group of users who used the feature and behavioral engagement of the number of users with respect to the feature. In the above medium, behavioral engagement of each of the number of users with respect to the application feature is indicated by a behavioral engagement metric (BEM) of each user of the number of users with respect to the application feature, wherein the BEM is based on an interaction of each user of the number of users with the feature, the interaction including at least one of: a frequency with which each user of the number of users interacts with the application feature; a frequency with which each user of the number of users ignores a guidance generated by the application feature; an average amount of time each user of the number of users spends interacting with the application feature; or how closely behavior of each user of the number of users adheres to the guidance generated by the application feature.

In the above medium, each of the one or more application features has a correlation with the objective that is above a correlation threshold. In the above medium, the method further comprises: receiving a plurality of inputs including: a first input including glucose measurements associated with the user generated by the glucose monitoring system; and a second input indicative of behavior of the user with respect to the one or more application features; calculating a programmatic outcome metric associated with the objective based at least on the first input, wherein the programmatic outcome metric is indicative of an extent to which the user has achieved the objective; calculating, based on the second input, one or more behavioral engagement metrics (BEMs) for the one or more application features, such that a separate BEM is calculated for each of the one or more application features; identifying one or more users in the selected group of users or the pool of users with BEMs similar to the calculated one or more BEMs; identifying a new application feature not included in the one or more features based on the feature being associated with a BEM above a threshold for at least one of the one or more users; and reconfiguring the application with the new application feature based on at least one of the one or more BEMs and the programmatic outcome metric.

In the above medium, each BEM of the one or more BEMs is based on an interaction of the user with a corresponding application feature of the one or more application features, the interaction including at least one of: a frequency with which the user interacts with the corresponding application feature; a frequency with which the user ignores a guidance generated by the corresponding application feature; an average amount of time the user spends interacting with the corresponding application feature; or how closely behavior of the user adheres to the guidance generated by the corresponding application feature.

In the above medium, reconfiguring the application with the new application feature comprises: identifying a low performing application feature of the one or more application features with a corresponding BEM that is below a threshold; and replacing the low performing application feature of the one or more application features with the new application feature. In the above medium, reconfiguring the application with the new application feature comprises: identifying that the low performing application feature of the one or more application features relates to the objective; and identifying that the programmatic outcome metric is below a threshold. In the above medium, each application feature of the one or more application features comprises a feature setting.

BRIEF DESCRIPTION OF THE DRAWINGS

FIG. 1A illustrates an example decision support system for selecting and adapting an application configuration of a diabetes intervention application that provides decision-support guidance to a user, according to some embodiments disclosed herein.

FIG. 1B illustrates the glucose monitoring system of FIG. 1A, in more detail, along with a number of mobile devices, according to some embodiments disclosed herein.

FIG. 2 illustrates example inputs and example metrics that are calculated based on the inputs for use by the decision support system of FIG. 1A, according to some embodiments disclosed herein.

FIG. 3 is a flow diagram illustrating operations performed by a system, such as the decision support system of FIG. 1A, according to some embodiments disclosed herein.

FIG. 4A is a diagram illustrative of how application configuration of an application is initially selected and then continuously adapted using a data model, according to some embodiments disclosed herein.

FIG. 4B illustrates a number of datasets used in the operation of the data model of FIG. 4A, according to some embodiments disclosed herein.

FIG. 5A is a diagram illustrative of how application configuration of an application is initially selected and then continuously adapted using a data model, according to some embodiments disclosed herein.

FIG. 5B illustrates a number of datasets used in the operation of the data model of FIG. 5A, according to some embodiments disclosed herein.

FIG. 6 is a block diagram depicting a computing device configured to perform the operations of FIG. 3, according to certain embodiments disclosed herein.

DETAILED DESCRIPTION OF CERTAIN INVENTIVE EMBODIMENTS

In certain embodiments, an application, as described herein, delivers guidance that may assist patients, caregivers, healthcare providers, or other users improve lifestyle or clinical/patient outcomes by meeting a variety of challenges, such as overnight glucose control (e.g., reduce incidence of hypoglycemic events or hyperglycemic excursions), glucose control during and after meals (e.g. use historical information and trends to increase glycemic control), hyperglycemia corrections (e.g., increase time in target zone while avoiding hypoglycemic events from over-correction), hypoglycemia treatments (e.g, address hypoglycemia while avoiding "rebound" hyperglycemia), exercise, and/or other health factors. In certain embodiments, the application may further be configured with optimization tools that learn a patient's physiology and behavior and calculate guidance to help the patient identify optimal or desirable therapy parameters, such as basal insulin requirements, insulin to carb ratios, correction factors, and/or changes to insulin sensitivity due to exercise.

The application may, for example, help a patient respond to a problem in real time by predicting hypoglycemia or hyperglycemia events or trends, providing treatment recommendations to address occurring or potential hypoglycemia or hyperglycemia events or trends, and/or monitor the patient's glycemic, physiologic, and/or behavioral response to different events in real time. This type of calculated guidance and support may relieve the cognitive burden on the user.

Physiologic sensors such as continuous glucose monitors can provide useful data that may be used by a user to manage glucose levels, but the data may require significant processing to develop effective strategies for glucose management. The sheer volume of data, and recognition of correlations between types of data, trends, events, and outcomes, can far exceed human capabilities for processing. This is particularly impactful when a decision about therapy or response to a physiologic condition is being made in real time. Integration of real-time or recent data with historical data and patterns can provide useful guidance in making real-time decisions about therapy. Technological tools can process this information to provide decision support guidance calculated to be useful for a particular patient in a particular condition or situation at a particular time.

When regularly used, an application could be a very powerful tool in managing a patient's diabetic condition. However, ongoing use of an application is directly correlated with whether the application's configuration helps the patient with achieving the patient's objectives on an ongoing basis. Certain existing applications suffer from a technical problem of failing to tailor or personalize an applications configuration based on the patient's specific objectives and behaviors. As such, since each patient's objectives and behaviors may be different from other patients, a static configuration that provides the same set of features to all patients on an ongoing basis poses a technical problem that renders such applications less likely to be used by the patient. As a result of this technical problem, these applications experience high attrition rates.

For example, a patient may download an application on the patient's mobile device with the intention of achieving certain objectives. However, after only days of use, the patient may decide that the features presented by the application are not very relevant to the patient in that they are not supporting the patient in meeting any of those objectives. As a result, the patient quickly loses interest in the application and drops its usage all together. In another example, a patient may initially use the application on a regular basis but as the patient's condition, objectives, and behavior change over time, the application may begin to feel irrelevant and useless to the patient, resulting in the patient losing interest in the application.

Accordingly, certain embodiments described herein provide a technical solution to the technical problem described above by providing decision support systems and methods for automatically configuring and adapting an application's configuration based on information relating to the specific user and/or information relating to a pool of users, such as a stratified group of users that are similar in one or more aspects to the user. In certain embodiments, an application configuration herein refers to a set of application features and their associated settings.

In certain embodiments, information relating to the specific user includes at least one of the user's objectives, interests, abilities, demographic information, disease progression, medication info, and/or a plurality of inputs and metrics, as further described below. In certain embodiments, the plurality of inputs and metrics include outcome and behavioral metrics. In certain embodiments, information relating to the specific user may include real-time information, historic information, and/or trends. In certain embodiments, information relating to a stratified group of users includes at least one of: the users' objectives, interests, abilities, demographic information, disease progression information, medication regimen information ("medication info"), a plurality of inputs and metrics associated with such users, and/or application configurations of the users' applications. In certain embodiments, information relating to a stratified group of users may include real-time information, historic information, and/or trends.

For example, after a user downloads the application, in certain embodiments, the decision support system may first work to identify certain information about the user. In certain embodiments, the information about the user includes the user's progression of a disease, medication information, demographic information, objectives, interests, and/or abilities. In certain embodiments, the decision support system may then identify a stratified group of users from a pool of users in a user database based on one or more stratification factors. In certain embodiments, the one or more stratification factors include the user's progression of a disease, medication information, demographic information, objectives, interests, and/or abilities. Information associated with the stratified group may provide indications that help with determining what application configuration may be helpful, such as the most helpful, to the user in achieving the user's objectives. Therefore, in certain embodiments, based on the information associated with the user, and/or information associated with the stratified group, the decision support system may select a certain application configuration and automatically configure the application with the selected application configuration. Subsequently, in certain embodiments, the decision support system may periodically monitor and evaluate one or more behavioral and outcome metrics associated with the user's use of the application and its selected application configuration. In certain embodiments, the decision support system then automatically adapts the application's configuration based on the user's behavioral metrics and/or information relating to other users in the user database. In certain embodiments, the other users are users who, for example, met the same objectives and exhibited similar behavioral metrics with respect to the use of the same application configuration. In certain embodiments, the decision support system continuously adapts the application's configuration.

As described above, in certain embodiments, to provide relevant and effective guidance, the application utilizes input from one or more physiological sensors, such as one or more analyte sensors. An example of an analyte sensor described herein is a glucose monitoring sensor that measures a concentration of glucose and/or a substance indicative of the concentration or presence of glucose and/or another analyte in the user's body. In some embodiments, the glucose monitoring sensor is a continuous glucose monitoring device, for example a subcutaneous, transdermal, transcutaneous, non-invasive, intraocular and/or intravascular (e.g., intravenous) device. In some embodiments, the device can analyze a plurality of intermittent blood samples. The glucose monitoring sensor can use any method of glucose measurement, including enzymatic, chemical, physical, electrochemical, optical, optochemical, fluorescence-based, spectrophotometric, spectroscopic (e.g., optical absorption spectroscopy, Raman spectroscopy, etc.), polarimetric, calorimetric, iontophoretic, radiometric, and the like.

The glucose monitoring sensor can use any known detection method, including invasive, minimally invasive, and non-invasive sensing techniques, to provide a data stream indicative of the concentration of the analyte in a host. The data stream is typically a raw data signal that is used to provide a useful value of the analyte to a user, such as a patient or health care professional (HCP, e.g., doctor, physician, nurse, caregiver), who may be using the sensor.

In some embodiments, the glucose monitoring sensor is an implantable sensor, such as described with reference to U.S. Pat. No. 6,001,067 and U.S. Patent Publication No. US-2011-0027127-A1. In some embodiments, the glucose monitoring sensor is a transcutaneous sensor, such as described with reference to U.S. Patent Publication No. US-2006-0020187-A1. In yet other embodiments, the glucose monitoring sensor is a dual electrode analyte sensor, such as described with reference to U.S. Patent Publication No. US-2009-0137887- A1. In still other embodiments, the glucose monitoring sensor is configured to be implanted in a host vessel or extracorporeally, such as the sensor described in U.S. Patent Publication No. US-20070027385- A1.

Although much of the description and examples below are drawn to a glucose monitoring sensor capable of measuring the concentration of glucose in a host, the systems and methods of the embodiments described herein can be used in conjunction with any type of analyte sensor for any measurable analyte. Also, although certain embodiments herein are described in relation to a diabetes intervention application, the system and methods of the embodiment described herein may be used in conjunction with any health-related application that is provided to the user to improve the user's health. For example, a health-related application may help the user with treating a certain disease or just help with improving the health of a user who is not necessarily diagnosed with a disease.

EXAMPLE SYSTEM

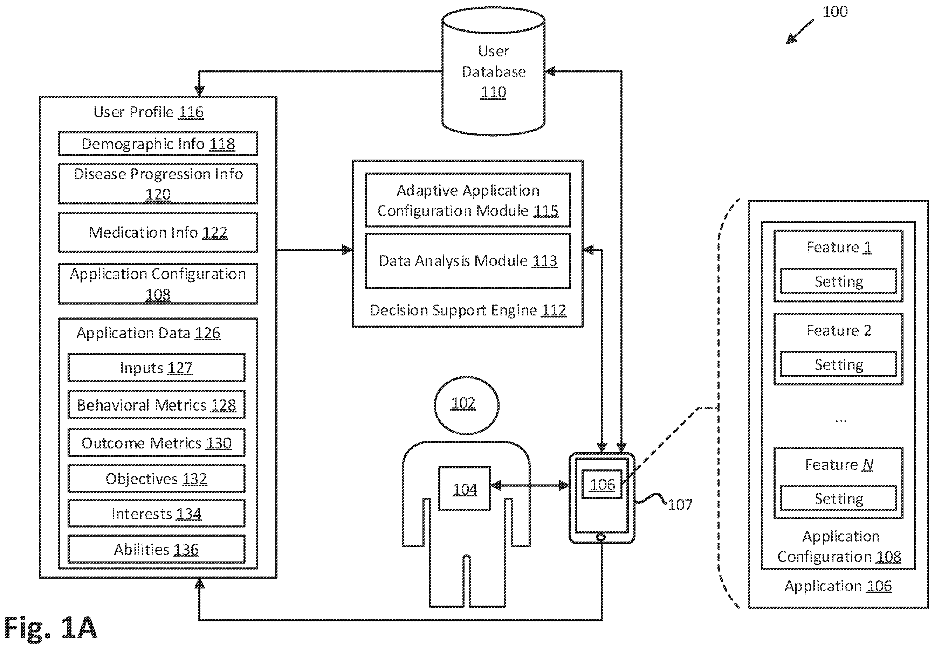

FIG. 1A illustrates an example decision support system 100 (also referred to as a. "health monitoring system") for selecting and adapting an application configuration of a diabetes intervention application ("application") 106 that provides decision-support guidance to user 102 (hereinafter "the user"), in certain embodiments. The user, m certain embodiments, may be the patient or the patient's caregiver. In the embodiments described herein, the user is assumed to be the patient for simplicity only but is not so limited. In certain embodiments, system 100 includes the user, a glucose monitoring system 104, a mobile device 107 that executes application 106, a decision support engine 112, and a user database 110. While die above system is described with respect to a glucose monitoring system, it should be rioted that this is exemplary, and one or more additional or alternative analytes may be monitored and used in accordance with the embodiments provided herein. In some embodiments, an analyte monitoring system (e.g., glucose monitoring system 104) is configured to measure at least one analyte selected from the group consisting of albumin, alkaline phosphatase, alanine transaminase, aspartate aminotransferase, bilirubin, blood urea nitrogen, calcium, carbon dioxide, chloride, creatinine, glucose, gamma-glutamyl transpeptidase, hematocrit, lactate, lactate dehydrogenase, magnesium, oxygen, pH, phosphorus, potassium, sodium, total protein, uric acid, a metabolic marker and a drug.

The term "analyte" as used herein is a broad term, and is to be given its ordinary and customary meaning to a person of ordinary skill in the art (and is not to be limited to a special or customized meaning), and refers without limitation to a substance or chemical constituent in a biological sample (e.g., bodily fluids, including blood, serum, plasma, interstitial fluid, cerebral spinal fluid, lymph fluid, ocular fluid, saliva, oral fluid, urine, excretions or exudates). Analytes can include naturally occurring substances, artificial substances, metabolites, and/or reaction products. In some embodiments, the analyte for measurement by the sensing regions, devices, and methods is albumin, alkaline phosphatase, alanine transaminase, aspartate aminotransferase, bilirubin, blood urea nitrogen, calcium, $CO_2$, chloride, creatinine, glucose, gamma-glutamyl transpeptidase, hematocrit, lactate, lactate dehydrogenase, magnesium, oxygen, pH, phosphorus, potassium, sodium, total protein, uric acid, metabolic markers, and drugs. However, other analytes are contemplated as well, including but not limited to acetaminophen, dopamine, ephedrine, terbutaline, ascorbate, uric acid, oxygen, d-amino acid oxidase, plasma amine oxidase, xanthine oxidase, NADPH oxidase, alcohol oxidase, alcohol dehydrogenase, pyruvate dehydrogenase, diols, Ros, NO, bilirubin, cholesterol, triglycerides, gentisic acid, ibuprophen, L-Dopa, methyl dopa, salicylates, tetracycline, tolazamide, tolbutamide, acarboxyprothrombin; acylcarnitine; adenine phosphoribosyl transferase; adenosine deaminase; albumin; alpha-fetoprotein; amino acid profiles (arginine (Krebs cycle), histidine/urocanic acid, homocysteine, phenylalanine/tyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, cystic fibrosis, Duchenne/Becker muscular dystrophy, glucose-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; glucose-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), B/A-1, β); lysozyme; mefloquine; netilmicin; phenobarbitone; phenyloin; phytanic/pristanic acid; progesterone; prolactin; prolidase; purine nucleoside phosphorylase; quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzi/rangeli, vesicular stomatis virus, Wuchereria bancrofti, yellow fever virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins, and hormones naturally occurring in blood or interstitial fluids can also constitute analytes in certain embodiments. The analyte can be naturally present in the biological fluid, for example, a metabolic product, a hormone, an antigen, an antibody, and the like. Alternatively, the analyte can be introduced into the body, for example, a contrast agent for imaging, a radioisotope, a chemical agent, a fluorocarbon-based synthetic blood, or a drug or pharmaceutical composition, including but not limited to insulin; ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbituates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine. The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-dihydroxyphenylacetic acid (DOPAC), homovanillic acid (HVA), 5-hydroxytryptamine (5HT), histamine, Advanced Glycation End Products (AGEs) and 5-hydroxyindoleacetic acid (FHfAA).

One aspect of the preferred embodiments provides an analyte monitoring system for in vivo continuous analyte monitoring (e.g,, albumin, alkaline phosphatase, alanine transaminase, aspartate aminotransferase, bilirubin, blood urea nitrogen, calcium, CO2, chloride, creatinine, glucose, gamma-glutamyl transpeptidase, hematocrit, lactate, lactate dehydrogenase, magnesium, oxygen, pH, phosphorus, potassium, sodium, total protein, uric acid, a metabolic marker, a drug, various minerals, various metabolites, and the like) that can be operatively coupled to a catheter to measure analyte concentration within the host's blood stream. In some embodiments, the system includes an analyte sensor that extends a short distance into the blood stream (e.g., out of the catheter) without substantially occluding the catheter or the host's blood stream. The catheter can be fluidly coupled to additional TV and diagnostic devices, such as a saline bag, an automated blood pressure monitor, or a blood chemistry monitor device. In some embodiments, blood samples can be removed from the host via the sensor system, as described elsewhere herein. In one embodiment, the analyte sensor is a glucose sensor, and the medical staff monitors the host's glucose level. In other embodiments, described elsewhere herein, the analyte sensor is disposed within or on the catheter itself, such as the in vivo portion of the catheter. In still other embodiments, the analyte sensor is disposed entirely within and/or on the fluid coupler, which is in turn fluidly coupled to a catheter or other vascular access device, as described elsewhere herein.

In certain embodiments, glucose monitoring system 104 includes a sensor electronics module and a glucose sensor that measures a concentration of blood glucose and/or a substance indicative of the concentration or presence of glucose and/or another analyte in the user's body. In certain embodiments, the glucose sensor is configured to perform measurements on a continuous basis. The sensor electronics module transmits the blood glucose measurements to mobile device 107 for use by application 106. In some embodiments, the sensor electronics module transmits the glucose measurements to mobile device 107 through a wireless connection (e.g., Bluetooth connection). In certain embodiments, mobile device 107 is a smart phone. However, in certain embodiments, mobile device 107 may instead be any other type of computing device such as a laptop computer, a smart watch, a tablet, or any other computing device capable of executing application 106.

In certain embodiments, decision support engine 112 refers to a set of software instructions with one or more software modules, including an adaptive application configuration module (AACM) 115 as well as a data analysis module (DAM) 113. In some embodiments, decision support engine 112 executes entirely on one or more computing devices in a private or a public cloud. In such embodiments, application 106 communicates with decision support engine 112 over a network (e.g., Internet). In some other embodiments, decision support engine 112 executes partially on one or more local devices, such as mobile device 107, and partially on one or more computing devices in a private or a public cloud. In some other embodiments, decision support engine 112 executes entirely on one or more local devices, such as mobile device 107.

As described below, in certain embodiments, AACM 115 is configured to adapt application configuration 108 of application 106 using information relating to the user, stored in user profile 116, and/or information relating to a pool of users, such as a stratified group of users, whose profiles may be stored in user database 110. In certain embodiments, DAM 113 is configured to process a set of inputs received from application 106 and compute a plurality of metrics, such as behavioral metrics 128 and outcome metrics 130, which can then be stored by application 106 in user profile 116. Behavioral metrics 128 and outcome metrics 130 may also be used by application 106, such as by different features of application 106, to provide real-time guidance to the user. The various data points of user profile 116, including behavioral metrics 128 and outcome metrics 130, are described in further detail below.

In certain embodiments, application 106 provides guidance to the user based on an adaptive application configuration 108. In certain embodiments, application configuration 108 comprises or refers to a set of features 1-*N*. In certain embodiments, the set of features may be selected from a larger pool of possible features, where *N* is meant to indicate that application configuration 108 may have any number of features. In certain embodiments, one or more of the features 1-*N* are configured to provide a form of guidance to the user to support the user in making more informed decisions. To provide effective, relevant, and on-time guidance to the user, in certain embodiments, a feature may take as input information relating to the user, stored in user profile 116, and/or information relating to a pool of users, stored in user profiles of such users in user database 110. In certain embodiments, a feature may interact with the user through various ways such as text, email, notifications (e.g., push notifications), phone calls, and/or other forms of communication such as displaying content (e.g., graphs, trends, charts, etc.) on the user interface of application 106. In certain embodiments, a feature also comprises a setting that defines the operations of the feature. In certain embodiments, changing a feature's setting results in changing the way the feature operates, such as the way the feature provides guidance and interacts with the user. For example, changing a feature's setting may result in one or more of, among other things, changing the frequency, content, timing, the form of the guidance provided by the feature, the guidance's time-frame (e.g., guidance can be real-time or retrospective/reflective), etc.

For example, in certain embodiments, features 1-*N* of application configuration 108 may include a number of different exercise management features. As an example, features 1, 2, 3, and 4 of application configuration 108 may correspond to exercise management feature 1 (e.g., a reminder feature for reminding the user about exercising at certain times), exercise management feature 2 (e.g., an exercise recommendation feature that recommends different types of exercise based on information relating to the user), exercise management feature 3 (e.g., a feature that calculates for how long the user should exercise to reach a certain blood glucose level), and exercise management feature 4 (e.g., a feature that recommends walking routes/hiking trails, etc.), respectively.

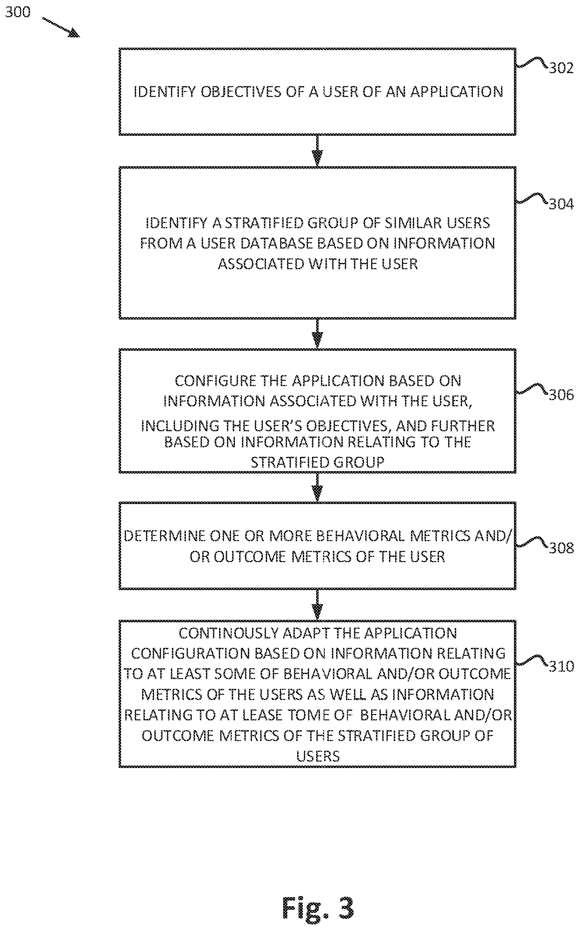

Further, in certain embodiments, an exercise management feature can be configured with a number of settings. As an example, exercise management feature 1 may configured with one of five settings (e.g., settings A, B, C, D, and E), where each setting configures exercise management feature 1 to provide guidance to the user with a different one or more of frequency, content, timing, form, time-frame, etc. For example, setting A may be the least aggressive or interactive (e.g., fewer reminders, different content, etc.) while setting E may be the most interactive. As such, in certain embodiments, application configuration 108 may comprise a plurality of feature and feature setting combinations ("FFSCs"), where each feature and feature setting combination corresponds to a certain feature (e.g., exercise management feature) with a certain setting (e.g., setting A). Defining features at this level of granularity may help identify which exercise management features and settings are useful to the user in meeting their objectives and which ones are not. For example, as further described in relation to FIG. 3, the user's behavioral metrics 128 may indicate that exercise management feature 1 with setting A is not useful to the user while information related to a stratified group of users may indicate that certain users who also found exercise management feature 1 with setting A not useful, instead found exercise management feature 1 with setting B very useful in helping them achieve their objectives. In such an example, AACM 115 may replace exercise management feature 1 with setting A with exercise management feature 1 with setting B. In contrast, if only a single exercise management feature with a single setting was defined, then it may be more difficult to identify what the user really finds useful about the feature and what aspects of the feature the user does not like to use. Additional example features are described in further detail below.

As described above, in certain embodiments, application 106 is configured to take as input information relating to the user and store the information in a user profile 116 of the user. For example, application 106 may obtain and record the user's demographic info 118, disease progression info 120, and/or medication info 122 in user profile 116. In certain embodiments, demographic info 118 may include one or more of the user's age, BMI (body mass index), ethnicity, gender, etc. In certain embodiments, disease progression info 120 may include information about the user's disease, such as whether the user is Type I, Type II, or pre-diabetes or whether the user has gestational diabetes. In certain embodiments, information about the user's disease may also include the length of time since diagnosis, the level of diabetes control, level of compliance with diabetes management therapy, predicted pancreatic function, other types of diagnosis (e.g., heart disease, obesity) or measures of health (e.g., heart rate, exercise, stress, sleep, etc.), and/or the like. In certain embodiments, medication regimen info 122 may include information about the amount and type of insulin or non-insulin diabetes medications and/or non-diabetes medication taken by the user.

In certain embodiments, application 106 may obtain demographic info 118, disease progression info 120, and/or medication info 122 from the user in the form of user input or from other sources. In certain embodiments, as some of this information changes, application 106 may receive updates from the user or other sources. In certain embodiments, user profiles, including user profile 116, are stored in a user database 110, which is accessible to application 106 as well as decision support engine 112 over one or more networks (not shown). User database 110, in some embodiments, refers to a storage server that may operate in a public or private cloud.

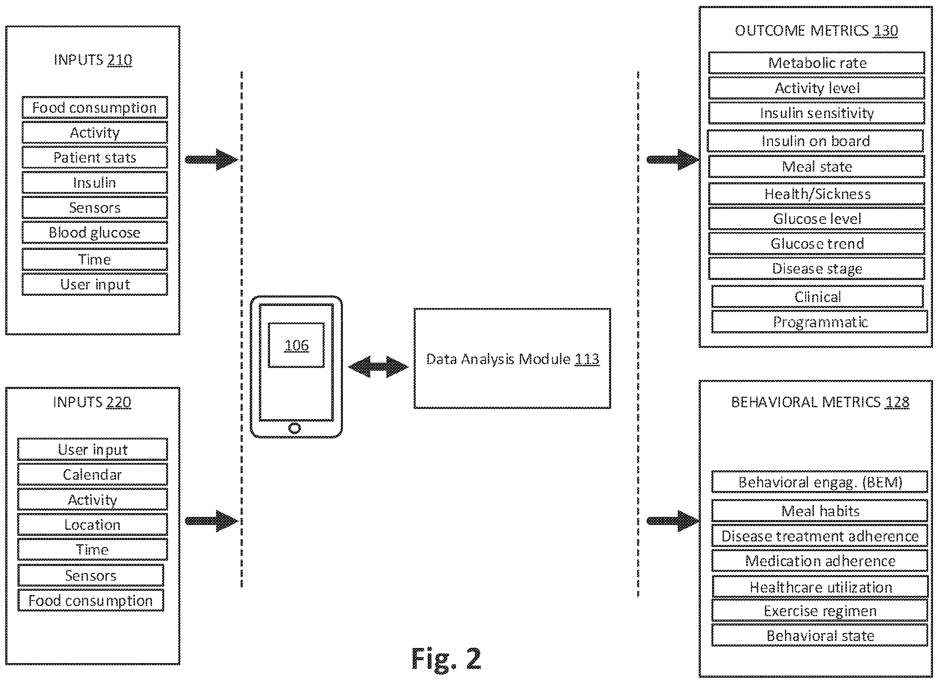

In certain embodiments, in addition to the user's demographic info 118, disease progression info 120, and/or medication info 122, application 106 obtains an additional set of inputs 127 that are also utilized by features 1-*N* to provide guidance to the user. In certain embodiments, such inputs 127 are obtained on a continuous basis. In certain embodiments, application 108 receives inputs 127 (e.g., including inputs 210 and 220 of FIG. 2) through user input and/or a plurality of other sources, including glucose monitoring system 104, other applications running on mobile device 107, and/or one or more other sensors and devices. In certain embodiments, such sensors and devices include one or more of, but are not limited to, an insulin pump, other types of analyte sensors, sensors or devices provided by mobile device 107 (e.g., accelerometer, camera, global positioning system (GPS), heart rate monitor, etc.) or other user accessories (e.g., a smart watch), or any other sensors or devices that provide relevant information about the user.

In certain embodiments, application 106 further uses at least part of inputs 127 to obtain a plurality of metrics, such as behavioral metrics 128 and outcome metrics 130. As further described in relation to FIG. 2, in some embodiments, application 106 transmits at least part of inputs 127 to DAM 113 for processing, based on which DAM 113 generates the plurality of metrics. In certain embodiments, behavioral metrics 128 and outcome metrics 130 may then be used by application 106 as input to features 1-*N* for providing guidance to the user. In certain embodiments, behavioral metrics 128 and outcome metrics 130 are also stored in user profile 116, which may be used by AACM 115 for adapting application configuration 108.

In certain embodiments, behavioral metrics 128 are a set of metrics that may be indicative of the user's behaviors and habits, such as one or more of the user's behavior or interaction with respect to the application, the user's behavior with respect to the treatment of the disease, health-related behaviors, etc. Behavioral metrics 128 may include real-time metrics, past metrics, and/or trends. Outcome metrics 130 may, at lease in some cases, be generally indicative of the user's health or state, such as one or more of the user's physiological or psychological state (e.g., stress level, happiness, etc.), trends associated with the user's health or state, how far or close the user is to meeting their objectives based on the user's health or state, etc. In certain embodiments, outcome metrics 130 may be directly correlated with the behavioral metrics 128, meaning that outcome metrics 130 may be the result of the user's behavior or pattern of behaviors. Examples of outcome metrics 130 include one or more of metrics associated with metabolic rates, glucose levels and trends, the user's health or sickness, etc. In certain embodiments, outcome metrics 130 may include real-time metrics, past metrics, and/or trends.

In certain embodiments, user profile 116 also includes user objectives 132, interests 134, and abilities 136, which, in certain embodiments, are either obtained by application 106 from the user in the form of user input or generated by application 106 based on information provided by the user, as described in relation to FIG. 3. In certain embodiments, objectives 132 relate to what the user intends to achieve with respect to improving their health, such as one or more of weight loss, meal choices, medication reminders, lowering A1c, medication dosing regimen, sleep, time-in-range, reducing hypoglycemia events, etc., as well as their psychological state. In certain embodiments, objectives 132 may be dynamic, meaning that the user may change their objectives over time. In some embodiments, objectives 132 are measurable metrics such that decision support engine 112 is able to determine whether objectives 132 are being met based on outcome metrics 130.

Further, in certain embodiments, using objectives 132, decision support engine 112 is able to track or determine the effectiveness of application configuration 108. For example, if the user is achieving or progressing towards achieving objectives 132, decision support engine 112 may take that as an indication that the features of application configuration 108 are at least somewhat helping the user. In contrast, if the user is not achieving or progressing towards achieving objectives 132, then that may indicate that application configuration 108 could be improved. In certain embodiments, interests 134 refer to the user's interests, such as the user's interests in one or more different activities, food, amount of sleep, certain types of therapies and/or medications, etc. In certain embodiments, abilities 136 refer to the user's abilities, such as the user's abilities to perform one or more of a possible pool of activities that may be recommended to the user in the form of guidance. Examples include one or more of physical disabilities, inability to eat certain foods, inability to engage in certain types of activities, inability to engage in certain activities during certain times but not others, etc.

In certain embodiments, user profile 116 is dynamic because at least part of the information that is stored in user profile 116 may be revised over time and/or new information may be added to user profile 116 by application 106. In certain embodiments, AACM 115 is configured to adapt application configuration 108 based on a dynamic user profile 116 and/or dynamic user profiles of a pool of users in user database 110 in order to provide guidance that helps the user achieve objectives 132. Adapting application configuration 108 may include changing (e.g., adding or removing) the set of features 1-*N* and/or changing settings of one or more of the features 1-*N*. In other words, in certain embodiments, each feature is also adaptive because the feature's setting can be adapted.

Although not limited to this list, some example features may include one or more of an objective setting and identification feature, reward features, reporting features, behavioral intervention features, medication reminder features, a glycemic impact estimator feature, an educational feature, etc.

As further described below, in certain embodiments, an objective setting and identification feature may guide the user through defining a set of objectives that the user may expect to achieve with help from application 106. In certain embodiments, reward features may provide rewards to the user to encourage healthy behavior.

In certain embodiments, reporting features may provide reports to the user in various forms. For example, a reporting feature may be configured to isolate and report glycemic excursions to the user so as to enable the user to think through the causes of such glycemic excursions. A report may be provided to the user in various forms, as one of ordinary skill in the art appreciates. For example, a reporting feature may display a graph of the user's glucose measurement trends with two glycemic excursions highlighted with text that says, "you had two high glucose events today; the first lasted for 55 minutes and the second lasted 30 minutes." In one example the reporting feature may provide an afternoon report that provides information about the user's blood glucose average so far for that day, indicate a rest-of-day average required to keep on track towards the user's objective, and recommend a low glycemic load or physical activity. In another example, the reporting feature may provide a nighttime summary that includes a daily, weekly, and monthly blood glucose averages, and estimated A1c. The summary informs the user of what the next day's blood glucose average must be in order for the user to attain their A1c goal. In certain embodiments, the summary is sent to the user at 8 PM and pushed again first thing in the morning if the user has not read it yet.

In certain embodiments, a reporting feature may be focused on providing the user with teachable moments. In certain embodiments, a teachable moment identifies the effect of behavior (e.g., physical activity, diet, medication adherence and sleep) on blood sugar. Teachable moments may be pushed to the user in the form of notification and/or recorded on a glucose monitoring curve to be reviewed in a timely manner by the user. For example, teachable moments may be visually displayed on the curve and describe the behavior and glucose response in order to inform the user as to what behavior caused what glucose response.

An example of a positive teaching moment may be provided when the user eats a high glycemic load breakfast that raises their blood sugar around 10AM. In certain embodiments, based on input from the user's accelerometer, the reporting feature may then determine that the user went for a 30-minute walk, which caused the user's blood glucose to return to their target range. In certain embodiments, the reporting feature marks this event as a teachable moment (e.g., by displaying a star on CGM curve) and may send a notification to the user, stating: "Nice walking, Sharon! Because of your 30-minute walk, your blood sugar is now right back where you want it."

In certain embodiments, behavioral intervention features include any feature that operates to change the user's behavior, such as by encouraging the user to engage in a certain behavior or to refrain from engaging in a certain behavior. As an example, a behavioral feature may be configured to send push notifications to the user to encourage the user to engage in a certain behavior. The behavioral feature may also be able to determine that a user is about to engage in a certain behavior, for example, based on the user's past behaviors, and send a push notification to the user to not engage in such behavior. In some embodiments, push notifications to the user may be based on information relating to the user (e.g., objectives 132, outcome metrics 130, behavioral metrics 128, etc.) and/or information relating to a stratified group of users. For example, the content of the push notification (e.g., the type or specifics of the behavior the push notification recommends) may be based on data relating to the types of behaviors that led to other users in the stratified group achieving the same objective.

For example, a type of a behavioral intervention feature may involve exercise management, for which one or more exercise management features may be provided. An example includes an exercise management feature that encourages the user, through a push notification, to exercise upon determining that the user's glucose levels are high or at a level where lack of exercise may cause the user's glucose levels to increase. In certain embodiments, the exercise management feature may be configured to receive and analyze data from an accelerometer, a global positioning system (GPS), a heartbeat monitoring sensor, glucose monitoring system 104, and/or other types of sensors and devices in order to provide more effective and tailored guidance to the user. For example, by receiving information from one or more of these sensors and devices, an exercise management feature may be able to make a determination as to whether one or more of the user actually engaged in an exercise, for how long the user should exercise to ensure the user's blood glucose gets back into normal range, what walking route should the user take, etc.

In certain embodiments, another type of a behavioral intervention feature may involve diet management for which one or more diet management features may be provided. For example, a diet management feature may act as a virtual dietician for providing guidance to the user as to one or more of when to eat, what to eat, how much to eat, etc. In certain embodiments, the diet management feature may provide personalized meal recommendations based on one or more of the user's real-time conditions (e.g., real-time blood glucose measurements), the user's body's response to certain meals, etc. In certain embodiments, the diet management feature may also help the user with meal prepping and/or shopping and/or allow the user to enter information about meals consumed by the user to understand nutritional values, etc. In certain embodiments, the diet management feature may further one or more of make menu and ingredient substitution suggestions at restaurants or suggest healthy restaurants and grocery stores within a certain geographical area. In certain embodiments, the diet management feature may also provide notifications based on information about the user's meal information. For example, if the user has had a meal and their blood glucose does not lower back into a target zone afterwards (e.g., the next pre-prandial peak (2 hours after initial meal-related glucose rise) is more than 180 mg/dL), then an urgent alert may be issued to the user to exercise immediately. However, if the user's last meal had a prost-prandial peak of less than 180 mg/dL, and pre-prandial glucose is in range (80-130), then the diet management feature may randomize if the user receives an alert after the next meal (e.g., reduce the likelihood of sending an alert by 33%). Note that the exercise and diet management features described above are merely two examples of behavioral intervention features.

In certain embodiments, the medication management features may provide notifications to the user for one or more of when the user needs to take medication, what type of medication (e.g., oral medication for Type II diabetic patients, and insulin injection for Type I diabetic patients, etc.) the user should take, in what dosage or amount, etc. A medication management feature may provide such notifications based on the user's specific information, such as one or more of the user's disease (e.g., Type I or Type II), current outcome metrics 116 (e.g., current blood levels and metrics), etc. In certain embodiments, the medication management feature may also one or more of keep track of how well the user has been following the medication schedule, order medication for the user automatically before the user runs out of the medication, and/or provide information about the medication itself (e.g., educate the user on the medication's impact and efficacy). For example, the medication management feature may query the patient as to whether they took their medication. If user answers "yes" three times in a row, then the medication management feature may randomize and assign a 33% chance for asking the user the next day. If the user does not answer "yes" three times in a row, then the medication management feature may send a reminder to the user to take their medication the next day.

In certain embodiments, the medication management feature may automatically communicate with an insulin administration device, such as a pump, to cause the device to administer the right dosage of insulin, based on the user's current outcome metrics 130. For example, the medication management feature may take into account the user's current glucose levels and metrics and determine an accurate amount of long-lasting insulin to be administered. In certain embodiments, the medication management feature may then send signals to the medicament administration device to administer the said amount of insulin. In certain embodiments, the medication management feature takes information relating to the stratified group of users into account when providing guidance to the user (e.g., when calculating the amount of insulin to be administered).

Other behavioral management features may include comorbid condition management features, obesity and weight management feature, and gender-specific features. Comorbid condition management features may include a cardiovascular health management feature (e.g., providing lactate guided exercise sessions for cardiovascular health, by replacing typical lab specific cardiopulmonary metrics), a liver health management feature (e.g., providing insight on the user's fasting lactate levels and lactate clearance rates), and a feature for managing glucose for users with chronic kidney disease (e.g., where A1C is not a good indicator of glycemic status, patients with renal disease can receive glucose derived metrics).

The obesity and weight management feature may provide (1) "fatmax" (point at which fat burning peaks) exercise guided training for weight management/weight loss through the use of lactate and/or glycerol measurements, (2) diet specific guidance for those interested in ketocyenic diets, and (3) post exercise nutrition specific guidelines guided by lactate measurements for users with Type 1 diabetes, Type 2 diabetes, and/or interest in health and wellness.

The gender-specific features include (1) a feature that provides glucose management guidance in relation to changes in menstrual cycle using sensor data from a temperature data, and (2) providing glucose management guidance to male users to reduce their maximal glucose concentrations and increase longevity.

In certain embodiments, the glycemic impact estimator feature may use mobile device 107's camera to scan a menu and convert each meal item into an estimated glycemic impact metric. In some embodiments, the glycemic impact estimator feature shows glycemic impact metrics superimposed over the menu items. In some embodiments, glycemic impact metrics are based on data from user profiles of a stratified group of users. In some embodiments, the glycemic impact estimator feature may highlight different menu items based on how healthy the items are using different coloring (e.g., green for healthier items and red for unhealthy items).

In certain embodiments, the educational feature educates the user about the user's condition and how the user can improve his/her health. In one example, the educational feature educates the user about the potential impact the user may see if the user adopts a certain lifestyle. In certain embodiments, to determine the potential impact, the educational feature may consider the impact other users in the stratified group, who adopted that same lifestyle, experienced. For example, the educational feature may state to the user: "by following this program, patients like you were able to lose 3 pounds or lower their A1C by 5% per month." In certain embodiments, the educational feature may also educate the user about the cause and effect of potential behaviors, such as based on the effects users in the stratified group experienced. In another example, the educational feature educates the user on topics that are selected and tailored based on the user's objectives 132, interest 134, and/or abilities 136.

FIG. IB illustrates glucose monitoring system 104 in more detail. FIG. IB also illustrates a number of mobile devices 107a, 107b, 107c, and .107d. Note that mobile device 107 of FIG. IA may beany one of mobile devices 107a, 107b, 107c, or 107d. In other words, any one of mobile devices 107a, 1 07b, 107c, or 107d may be configured to execute application 106. Glucose monitoring system 104 may be communicatively coupled to mobile devices 107a, 107b, 107c, and/or 107d.

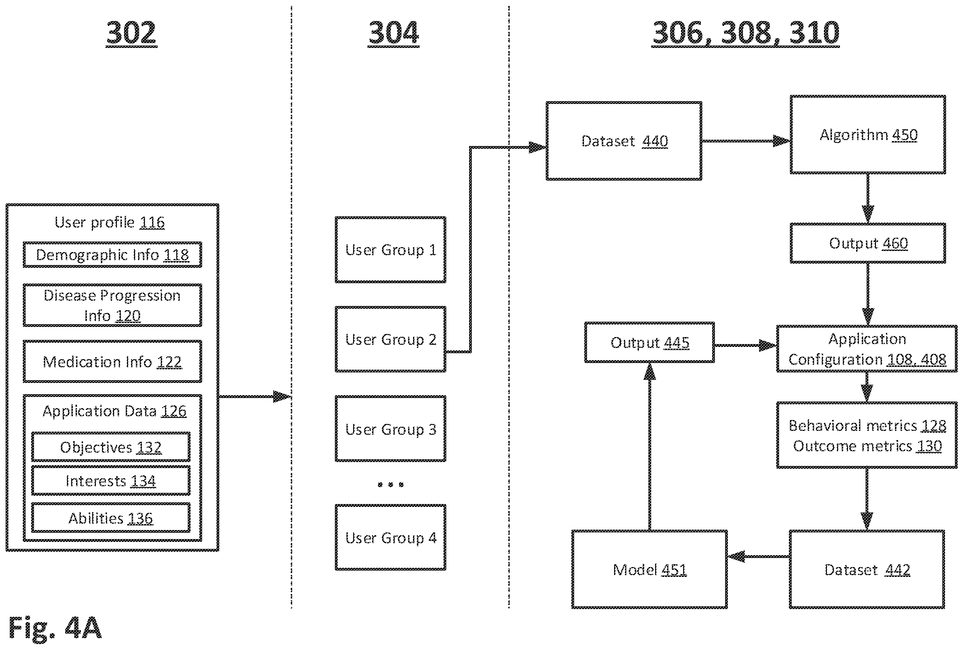

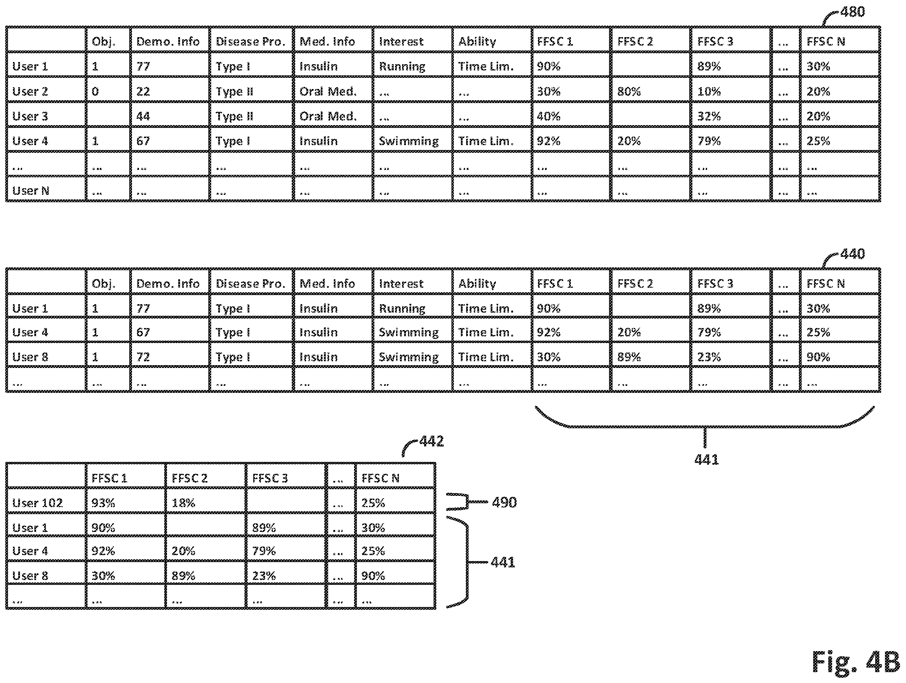

By way of an overview and an example, glucose monitoring system 104 may be implemented as an encapsulated microcontroller that makes sensor measurements, generates analyte data (e.g., by calculating values for continuous glucose monitoring data), and engages m wireless communications (e.g., via Bluetooth and/or other wireless protocols) to send such data to remote devices, such as mobile devices 107a, 107b, 107c, and/or 107d. Paragraphs [0137]-[0l40] and FIGs. 3A, 3B, and 4 of U.S. App. No. 2019/0336053 further describe an on-skin sensor assembly that, in certain embodiments, may be used in connection with glucose monitoring system 104. Paragraphs [0137]-[0140] and FIGs. 3 A, 3B, and 4 of U.S. App. No. 2019/0336053.

In certain embodiments, glucose monitoring system 104 includes an analyte sensor electronics module 138 and a glucose sensor 140 associated with analyte sensor electronics module 138. In certain embodiments, analyte sensor electronics module 138 includes electronic circuitry7 associated with measuring and processing analyte sensor data or information, including algorithms associated with processing and/or calibration of the analyte sensor data/information. Analyte sensor electronics module 138 may be physically/'mechanically connected to glucose sensor 140 and can be integral with (i.e., non-releasably attached to) or releasably attachable to glucose sensor 140.

Analyte sensor electronics module 138 may also be electrically coupled to glucose sensor 140, such that the components may be electromechanically coupled to one another. Analyte sensor electronics module 138 may include hardware, firmware, and/or software that enable measurement and/or estimation of levels of the analyte in the user via glucose sensor 140 (e.g., which may be/include a glucose sensor). For example, analyte sensor electronics module 138 can include one or more potentiostats, a power source for providing power to glucose sensor 140, other components useful for signal processing and data storage, and a telemetry module for transmitting data from the sensor electronics module to one or more display devices. Electronics can be affixed to a printed circuit board (PCB) within glucose monitoring system 104, or platform or the like, and can take a variety of forms. For example, the electronics can take the form of an integrated circuit (IC), such as an Application-Specific Integrated Circuit (ASIC), a microcontroller, a processor, and/or astate machine.

Analyte sensor electronics module 138 may include sensor electronics that are configured to process sensor information, such as sensor data, and generate transformed sensor data and displayable sensor information. Examples of systems and methods for processing sensor analyte data are described in more detail herein and in U.S. Pat. Nos. 7,310,544 and 6,931,327 and U.S. Patent Publication Nos. 2005/0043598. 2007/0032706, 2007/0016381, 2008/0033254, 2005/0203360, 2005/0154271, 2005/0192557, 2006/0222566, 2007/0203966 and 2007/0208245.

Glucose sensor 140 is configured to measure a concentration or level of the analyte in the user 102. The term analyte is further defined by paragraph [0117] of U.S. App. No. 2019/0336053. Paragraph [0117] of U.S. App. No. 2019/0336053. Tn some embodiments, glucose sensor 140 comprises a continuous glucose sensor, such as a subcutaneous, transdermal (e.g., transcutaneous), or intravascular device. In some embodiments, glucose sensor 140 can analyze a plurality of intermittent blood samples. Glucose sensor 140 can use any method of glucose-measurement, including enzymatic, chemical, physical, electrochemical, spectrophotometric, polarimetric, calorimetric, iontophoretic, radiometric, immunochemical, and the like. Additional details relating to a continuous glucose sensor are provided in paragraphs [0072]-[0076] of U.S. App. No.13/827,577. Paragraphs [0072]-[0076] of U.S. App. No. 13/827,577.

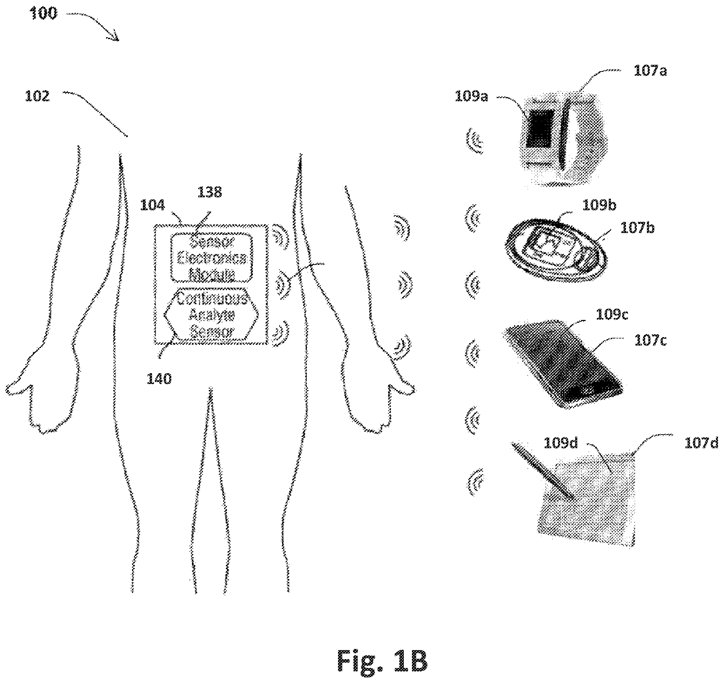

With further reference to FIG. 1B, mobile devices 107a, 107b, 107c, and/or 107d can be configured for displaying (and/or alarming) displayable sensor information that may be transmitted by sensor electronics module 138 (e.g., in a customized data package that is transmitted to the display devices based on their respective preferences). Each of mobile devices 107a, 107b, 107c, and/or 107d may respectively include a display such as touchscreen display 109a, 109b, 109c, and/or 109d for displaying a graphical user interface of application 106 for presenting sensor information and/or analyte data to user 102 and/or receiving inputs from user 102. In certain embodiments, the mobile devices may include other types of user interfaces such as voice user interface instead of or in addition to a touchscreen display for communicating sensor information to user 102 of the mobile device and/or receiving user inputs. In certain embodiments, one, some, or all of mobile devices 107a, 107b, 107c, and/or 107d may be configured to display or otherwise communicate the sensor information as it is communicated from sensor electronics module 138 (e.g., in a data package that is transmitted to respective display devices), without any additional prospective processing required for calibration and/or real-time display of the sensor data.

The plurality of mobile devices 107a, 107b, 107c, and/or 107d depicted in FIG. 1B may include a custom or proprietary display device, for example, analyte display device 107b, especially designed for displaying certain types of displayable sensor information associated with analyte data received from sensor electronics module 138 (e.g., a numerical value and/or an arrow, in certain embodiments). In certain embodiments, one of the plurality of mobile devices 107a, 107b, 107c, and/or 107d includes a smartphone, such as mobile phone 107c, based on an Android, iOS, or another operating system configured to display a graphical representation of the continuous sensor data (e.g., including current and/or historic data).

FIG. 2 provides a more detailed illustration of example inputs and example metrics that are determined based on the inputs, in accordance with certain embodiments. FIG. 2 illustrates example inputs 210 and 220 on the left, application 106 and DAM 113 in the middle, and outcome metrics 130 and behavioral metrics 128 on the right. Application 106 obtains inputs 210 and 220, which are part of inputs 127, through one or more channels (e.g., manual user input, sensors, other applications executing on mobile device 107, etc.). In certain embodiments, inputs 210 and 220 may be used by features 1-$N$ of application 106 to provide guidance to the user. Inputs 210 and 220 may also be further processed by DAM 113 to output a plurality of metrics, such as outcome metrics 130 and behavioral metrics 128, which may similarly be used by features 1-$N$ of application 106 to provide guidance to the user.

As described above, in certain embodiments, outcome metrics 130 and behavioral metrics 128 are also used by AACM 115 to adapt application configuration 108. In one example, inputs 210 are used by DAM 113 to output outcome metrics 130 while inputs 220 are used to output behavioral metrics 128, However, in other examples, any of inputs 210 and 220 may be used for computing any of the outcome metrics 130 and behavioral metrics 128. In certain embodiments, each metric may correspond to one or more values, e.g., discrete numerical values, ranges, or qualitative values (high/-medium/low or stable/unstable.).

In certain embodiments, starting with inputs 210, food consumption information may include information about one or more of meals, snacks, and/or beverages, such as one or more of the size, content (carbohydrate, fat, protein, etc.), sequence of consumption, and time of consumption. In certain embodiments, food consumption may be provided by a user through manual entry, by providing a photograph through an application that is configured to recognize food types and quantities, and/or by scanning a bar code or menu. In various examples, meal size may be manually entered as one or more of calories, quantity ('three cookies'), menu items ('Royale with Cheese'), and/or food exchanges (1 fruit, 1 dairy). In some examples, meals may also be entered with the user's typical items or combinations for this time or context (e.g., workday breakfast at home, weekend brunch at restaurant). In some examples, meal information may be received via a convenient user interface provided by application 106.

In certain embodiments, activity information is also provided as an input. Activity information may be provided, for example, by an accelerometer sensor on a wearable device such as a watch, fitness tracker, and/or patch. In certain embodiments, activity information may also be provided through manual user input.

In certain embodiments, patient statistics, such as one or more of age, height, weight, body mass index, body composition (e.g., % body fat), stature, build, or other information may also be provided. In certain embodiments, patient statistics are provided through a user interface, by interfacing with an electronic source such as an electronic medical record, and/or from measurement devices. In certain embodiments, the measurement devices include one or more of a wireless, e.g., Bluetooth-enabled, weight scale and/or camera, which may, for example, communicate with the mobile device 107 to provide patient data.

In certain embodiments, input relating to the patient's insulin delivery may be received, via a wireless connection on a smart pen, via user input, and/or from an insulin pump. Insulin delivery information may include one or more of insulin volume, time of delivery, etc. Other parameters, such as insulin action time or duration of insulin action, may also be received as inputs.

In certain embodiments, input may also be received from sensors, such as physiologic sensors, which may detect one or more of heart rate, respiration, oxygen saturation, or body temperature (e.g. to detect illness). In certain embodiments, electromagnetic sensors may also detect low-power RF fields emitted from objects or tools touching or near the object, which may provide information about the patient activity or location. An example of information that can be received from sensors is the user's blood glucose values.

In certain embodiments, blood glucose information may also be provided as input, for example through a glucose monitoring system 104. In certain embodiments, blood glucose information may be received from one or more of smart pill dispensers that track when the user takes medicine, a blood ketone meter, a laboratory-measured or estimated AIC, other measures of long-term control, or sensors that measure peripheral neuropathy using tactile response, such as by using haptic features of a smartphone, or a specialty device.

In certain embodiments, time may also be provided as an input, such as time of day, or time from a real-time clock.

User input through a user interface, such a user interface of mobile device 107, may include any other types of inputs a user may provide to application 106, such as the other types of inputs 210 mentioned above. For example, in certain embodiments, user input may include one or more of mental state or stressor information, the delivery of therapy, such as the use of glucagon to stimulate liver release of glycogen in response to a low blood sugar, recommended basal rates or insulin-to-carb ratios (e.g. received from a clinician), recorded activity (e.g. intensity, duration and time completed or started), etc. In certain embodiments, the user input may also indicate medication intake (e.g., type and dosage of medication as well as the timing of when medication is taken).

As described above, in certain embodiments, DAM 113 determines or computes the user's outcome metrics 130 based on inputs 210. An example list of outcome metrics 130 is shown in FIG. 2.

In certain embodiments, metabolic rate is a metric that may indicate or include a basal metabolic rate (e.g., energy consumed at rest) and/or an active metabolism, e.g., energy consumed by activity, such as exercise or exertion. In some examples, basal metabolic rate and active metabolism may be tracked as separate outcome metrics. In certain embodiments, the metabolic rate may be calculated by DAM 113 based on one or more of inputs 210, such as one or more of activity information, sensor input, time, user input, etc.

In certain embodiments, the activity level metric may indicate the user's level of activity. In certain embodiments, the activity level metric be determined, for example based on input from an activity sensor or other physiologic sensors. In certain embodiments, the activity level metric may be calculated by DAM 113 based on one or more of inputs 210, such as one or more of activity information, sensor input, time, user input, etc.

In certain embodiments, the insulin sensitivity metric may be determined using historical data, real-time data, or a combination thereof, and may, for example, be based upon one or more inputs 210, such as one or more of food consumption information, blood glucose information, insulin delivery information, the resulting glucose levels, etc. In certain embodiments, the insulin on board metric may be determined using insulin delivery information, and/or known or learned (e.g. from patient data) insulin time action profiles, which may account for both basal metabolic rate (e.g., update of insulin to maintain operation of the body) and insulin usage driven by activity or food consumption.

In certain embodiments, the meal state metric may indicate the state the user is in with respect to food consumption, For example, the meal state may indicate whether the user is in one of a fasting state, pre-meal state, eating state, post-meal response state, or stable state. In certain embodiments, the meal state may also indicate nourishment on board, e.g., meals, snacks, or beverages consumed, and may be determined, for example from food consumption information, time of meal information, and/or digestive rate information, which may be correlated to food type, quantity, and/or sequence (e.g., which food/beverage was eaten first.).

In certain embodiments, health and sickness metrics may be determined, for example, based on one or more of user input (e.g., pregnancy information or known sickness information), from physiologic sensors (e.g., temperature), activity sensors, or a combination thereof. In certain embodiments, based on the values of the health and sickness metrics, for example, the user's state may be defined as being one or more of healthy, ill, rested, or exhausted.

In certain embodiments, glucose level metrics may be determined from sensor information (e.g., blood glucose information obtained from glucose monitoring system 104). In some examples, a glucose level metric may also be determined, for example, based upon historical information about glucose levels in particular situations, e.g., given a combination of food consumption, insulin, and/or activity. In certain embodiments, a blood glucose trend may be determined based on the glucose level over a certain period of time.

In certain embodiments, outcome metrics also include a disease stage, such as for Type II diabetics. Example disease stages for Type II diabetics can include a prediabetic stage, an oral treatment stage, and a basal insulin treatment stage. In certain embodiments, degree of glycemic control (not shown) may also be determined as an outcome metric, and may be based, for example, on one or more of glucose levels, variation in glucose level, or insulin dosing patterns.

In certain embodiments, clinical metrics generally indicate the clinical state the user is in with respect to the user's one or more conditions, such as diabetes. For example, in the case of diabetes, clinical metrics may be determined based on glycemic measurements, including one or more of Al c, trends in A1c, time in range, time spent below a threshold level, time spent above a threshold level, and/or other metrics derived from blood glucose values. In certain embodiments, clinical metrics may also include one or more of estimated A1c, glycemic variability, hypoglycemia, and/or health indicator (time magnitude out of target zone).

In certain embodiments, programmatic outcomes are metrics that are calculated to determine how successful the user is in meeting their defined objectives, as further described below. For example, DAM 113 may take as input any relevant one or more outcome metrics mentioned above and then determine how far or close the user is to achieving a corresponding objective, which may also be defined in terms of concrete metrics. In certain embodiments, whether an outcome metric is relevant depends on the user's objectives. For example, if the user's objective is to maintain an A1c of 5%, then metrics such as one or more of glucose level, glucose trends, clinical metrics, etc., may be used to determine whether the user is meeting that objective or not. In certain embodiments, DAM 113 may also use inputs 210 to determine whether the user is meeting their objectives. For example, if the user's objective is to lose 5 pounds, then user input relating to the user's weight can be used to determine a programmatic outcome metric that indicates whether the user has lost 5 pounds. In one example, programmatic outcome metrics may be quantified using percentages. For example, if the user in the example above loses 3 pounds, then the user's programmatic outcome metric with respect to that objective may be 60% (3/5). In certain embodiments, DAM 113 may also evaluate trends with respect to inputs 210 and outcome metrics 130 to determine progression of the user towards achieving the objective (i.e., the whether the user is moving in the right direction) with respect to achieving an objective, even though the user may not have achieved the objective yet.

FIG. 2 also illustrates behavioral metrics that are determined based on inputs 220. In certain embodiments, inputs 220 include user input, such as input through a user inter-face. In certain embodiments, user input, or lack thereof, may indicate the user's level of interest in a certain FFSC. The level of interest in a FFSC may indicate how helpful the user believes the FFSC is in helping the user achieve their objectives. For example, a low user interest may be recorded for a certain FFSC, if the user ignores a certain reminder of the FFSC, does not input information that is requested by the FFSC, indicates specifically that the user does not like a certain form of guidance or interaction provided by the FFSC, etc. In certain embodiments, the user input may also indicate medication intake (e.g., type and dosage of medication and/or the timing of when medication is taken). In certain embodiments, inputs 220 also include calendar information, such as availability or activity information received from a computer or smartphone calendar application executing on mobile device 107. In certain embodiments, inputs 220 also include activity information, as described above. In certain embodiments, inputs 220 also include information about the location (e.g., GPS data) of the user and/or time (e.g., from a real-time clock). Input from sensors as well as input relating to the user's food consumption are described above. In certain embodiments, additional inputs are also possible.

As described above, in certain embodiments, DAM 113 determines or computes the user's behavioral metrics 128 based on inputs 210. An example list of behavioral metrics 128 is shown in FIG. 2. Behavioral metrics include one or more of behavioral engagement metrics and/or metrics relating to one or more of meal habits, disease treatment adherence, medication type and adherence, healthcare utilization, exercise regimen, behavioral state, etc.

In certain embodiments, a behavioral engagement metric (BEM) indicates the level of engagement relating to the user's interaction with a certain FFSC of application 106. In certain embodiments, a BEM may be calculated for each FFSC in application configuration 108, as further described below. In certain embodiments, a BEM associated with a certain FFSC may be calculated based on the user's interaction with the feature, which is indicated by the user input provided as part of inputs 220, and/or other inputs such as time and calendar (e.g., to calculate frequency of interaction, etc.). For example, a BEM for a certain FFSC may be calculated based on information such as one or more of the frequency with which the user interacts with the FFSC, the frequency with which the user ignores a guidance generated by the FFSC, the average amount of time the user spends interacting with the FFSC, etc. In some embodiments, the frequency with which the user interacts with the FFSC or other time-related data points may be calculated based on, for example, an interaction log that keeps records of one or more of each time the user interacts with the feature, the amount of time the user spends interacting with the feature, etc. In certain embodiments, a BEM calculated for a FFSC may also take into account the consistency of the user's behavior towards the FFSC. In certain embodiments, a BEM calculated for a FFSC may also take into account the reliability of the user's behavior towards the FFSC. Behavioral reliability may relate to the level of confidence or certainty in the user's behavior towards the FFSC.

In certain embodiments, BEMs may also be based on other behavioral metrics such as one or more of meal habits, medication adherence, blood glucose data, etc., because these other behavioral metrics may be indicative of how engaged the user is with application 106. As an example, metrics relating to the user's meal habits may be taken into account to determine the user's BEM towards a diet management related FFSC. For example, if the user is not consuming the types of meals recommended by a diet management FFSC, which can be determined based on food consumption information, then the user's BEM towards the diet management FFSC may be lowered. In certain embodiments, a BEM for a diet management related FFSC, $FFSC_x$ may be calculated as:

$$BEM_{FFSC_x} = (W_1)(\text{User Interaction}) + (W_2)(MH) + \dots$$

In the formula above, $W_1$ is a defined weight that may be assigned to the user's interaction with $FFSC_x$. $W_2$ is a defined weight that may be assigned to the user's meal habit metric. Additional and/or alternative metrics may be considered or included in the formula above for calculating the BEM metric for $FFSC_x$. BEMs for other FFSCs may be similarly calculated not only based on the user's interaction with the FFSC, but, in certain embodiments, also based on other relevant behavioral metrics.

In certain embodiments, meal habits are measured by one or more metrics based on the content and the timing of the user's meals. For example, if a meal habit metric is on a scale of 0 to 1, the better/healthier meals the user eats the higher the meal habit metric of the user will be to 1, in an example. Also, the more the user's food consumption adheres to a certain time schedule, the closer their meal habit metric will be to 1, in the example. In certain embodiments, disease treatment and adherence are measured by one or more metrics that are indicative of how committed the user is towards treating the user's disease. In certain embodiments, disease treatment and adherence metrics are calculated based on one or more of the user's diet or food consumption, exercise regimen, medication adherence, etc. In certain embodiments, medication adherence is measured by one or more metrics that are indicative of how committed the user is towards their medication regimen. In certain embodiments, medication adherence metrics are calculated based on one or more of the timing of when the user takes medication (e.g., whether the user is on time or on schedule), the type of medication (e.g., is the user taking the right type of medication), and the dosage of the medication (e.g., is the user taking the right dosage).

In certain embodiments, healthcare utilization is measured by one or more metrics that are indicative of how often the user utilizes the pharmacy or visits a medical professional. In certain embodiments, healthcare utilization metrics are calculated based on one or more of user input, which includes one or more of information about user's prescriptions, pharmacy visits, visits to a doctor's office, etc., as well as location input, calendar input, etc.. In certain embodiments, exercise regimen is measured by one or more metrics that are indicative of one or more of what type of activities the user engages in, how intense the activities are, how often the user engages in such activities, etc. In certain embodiments, the exercise regimen metrics may be calculated based on one or more activity sensors, calendar input, user input, etc. In certain embodiments, the behavioral state of the user refers to one or more metrics for measuring the current behavior of the user (e.g., sleep state (e.g. sleep or resting or awake, which may be inferred from an activity sensor, calendar, or other information), appetite (which may be inferred for example from meal patterns), etc.).

FIG. 3 is a flow diagram illustrating example operations 300 performed by a system (e.g., system 100) for selecting an application's configuration and adapting it based on information relating to a user and/or information relating to a pool of users, such as a stratified group of users that are similar in one or more aspects to the user. Operations 300 are described below with reference to FIGs. 1 and 2 and their components.

At step 302, operations 300 begin by identifying objectives of a user of application 106. Step 302 may be performed by application 106, in some embodiments. In one example, application 106 may identify objectives 132 of the user during the initial set-up process, such as when the user first downloads the application. In another example, application 106 may identify objectives 132 ("objectives") of the user at a later time. In such an example, the user may use application 106 for a period of time and then decide to interact with application 106 to set some objectives. As described above, in certain embodiments, application 106 may use an objective setting and identification feature (OSIF) that is configured to identify the user's objectives through some sort of interaction with the user.

In certain embodiments, by identifying the user's objective, application 106 is able to determine what is really important to the user and how the user defines success. In some embodiments, the OSIF may query the user to identify the objectives by providing the user with a user-interface that allows the user to input their objectives. The user interface may, for example, include a drop-down menu with some potential objectives from which the user can select. For example, the drop down menu may include options such as a) "I would like to lose weight," b) "I'd like to sleep better at night," c) "I'd like to better manage my blood glucose levels at night," d) "I'd like to improve my meal choices," e) "I'd like to optimize my dosing regimen," f) "I'd like to improve my time in range," etc. As one of ordinary skill in the art appreciates, a variety of other interactive user-interface options may be used for obtaining the user's input.

In some embodiments, the OSIF may obtain the user's objectives by having a dialogue with the user. In one example, the OSIF may have a dialogue with the user using a chat bot. In another example, the OSFI may interact with the user through voice by asking the user what the user's objectives are. In such embodiments, the user's responses may be processed and recorded as text using a speech recognition feature. The text may then be processed using a natural language processing feature to derive one or more objectives. In some examples, the OSIF may pose questions to the user that directly inquire about the user's objectives, such as the following: "tell me what your objectives are." In other examples, the OSIF may engage in a more casual conversation with the user and infer the user's objectives from the user's responses. For example, the OSIF may start a conversation with the user by stating: "tell me what your diabetes management would look like in a perfect world?" or "how would you measure what you are doing with your diabetes?" In such examples, the OSIF infers the objectives from the user's responses.

In some examples, the user's input with respect to his/her objectives may be very specific and quantifiable. As an example, a user may define an objective as "lowering my A1c by two full points." In other examples, the OSIF may allow the user to define objectives in a less specific and more qualitative format to make the objective identification process easier for the user. For example, the OSIF may provide the user with the following options: "I'd like to strive for 'normal' glucose control," or "I'd like to strive for aggressive glucose control," etc. In an example, in response, the user may select the first option, in which, the OSIF may examine a set of guidelines that application 106 may be configured with to convert or translate "normal" glucose control into a concrete and quantifiable objective. As an example, if the OSIF has determined that the user is pregnant, then "normal" glucose control may be translated into an A1c of 5%. In another example, the OSIF may translate "normal" glucose control into a concrete objective by examining information relating to a stratified group of user. In such an example, this translation may be performed after a stratified group of users have been selected at step 304.

In some embodiments, the user's input with respect to their objectives may be relative to the stratified group of users. For example, the user may select or state: "I'd like to be in the top third of my peers with respect to, for example, glucose control, health, weight, etc." In such an example, the OSIF may similarly translate that statement into concrete and quantifiable objectives that are defined in the form of metrics based on examining information relating to a stratified group of user, after a stratified group of users have been selected at step 304. For instance, the OSIF may translate "being in the top third of my peers" to a set of specific metrics (e.g., an A1c of 6%, a weight of 180 pounds, etc.) and a set of possible therapies or action plans. The possible set of therapies may include (a) consuming 25% less carbs and exercising an additional 20 minutes three time a week, (b) taking additional or different medication (e.g., seeing a physician to add additional daily oral medication), (c) following a particular diet regimen that reduces carbs below a certain amount per day, etc. In certain embodiments, the OSIF may then present these possible sets of therapies and let the user choose one of the therapies, which clearly define, in quantifiable metrics, what is expected of the user to, for example, be in the top third of their peers. In certain embodiments, the OSIF may also allow the user to fine-tune a therapy after selecting it.

Note that the OSIF may follow a similar process even in scenarios where the user's non-specific input is not defined relative to a stratified group of users. "Normal glucose control," "good health," "healthy zone," are examples of non-specific user input that are not relative to a stratified group. After receiving such user inputs, the OSIF may examine information relating to a stratified group of users to translate such non-specific user inputs into a set of specific metrics and a set of possible therapies.

In certain embodiments, the OSIF may determine a set of specific objectives (in the form of metrics) that the user should strive to achieve based on a set of inputs (e.g., inputs 127) associated with the user. In certain embodiments, these inputs may already be available during the set-up process of application 106. In such embodiments, the objectives 132 that are set during step 302 are based on such inputs (e.g., for a user who has been using application 106 prior to using the OSIF). In certain embodiments, such inputs may not be available during the set-up process. As such, an initial set of objectives may be set for the user but as inputs becomes available for the user, during the user's use of application 106, the OSIF may revise the initial objectives based on the user's own inputs. For example, initially, during the set-up process, the user's A1C level may be at X% and the OSIF may suggest an objective of 0.9X%. However, as the user uses application 106, the OSIF may determine that the user's A1C level is in fact at 0.85X% and therefore adjust the objective to 0.8X%. In another example, as the user uses application 106, the OSIF may determine that the user's A1C level is in fact at 1.2X% and therefore adjust the objective to X%, based on a determination that 0.9X% may not be achievable for the user. In yet another example, as the user uses application 106, the OSIF may determine that the user's A1C level is X% but that similar users in the stratified group of users have an average A1C level of 0.7X%. In such an example, the OSIF may adjust the user's initial objective of 0.9X% to 0.8X%.

In some embodiments, objectives may be categorized into short-term and long-term objectives. An example of a short-term objective is "cutting the medication dose and cost in half." An example of a long-term objective is "cutting medication out altogether."

In some embodiments, the OSIF further identifies the user's interests 134 and abilities 136 through the same types of interactions described above. Identifying the user's interests 134 and abilities 136 is advantageous because application configuration 108 may be configured and adapted by AACM 115 based on not only the user's objectives 132, but also the user's interest 134 and abilities 136. As an example, if the user indicates that they are disabled and cannot walk, an exercise management feature of application 106 may be configured to suggest exercises or activities to the user that will not require the user to walk or run. In one example, the OSIF may query the user about the user's interests and abilities based on the FFSCs that are available by application 106. For example, it may not be relevant whether the user has interest in playing chess because, in certain embodiments, application 106 does not provide a feature that benefits from that input. However, asking whether the user likes to swim or hike may be more relevant because, in certain embodiments, application 106 actually provides exercise management features, which benefit from the user's response as to whether they like to swim.

At step 304, operations 300 continue by identifying a stratified group of similar users from a user database based on information associated with the user. Step 304 may be performed by AACM 115, in some embodiments. For example, AACM 115 may retrieve user profile 116 from user database 110 and select a stratified group of users from user database 110 based on information in user profile 116. In certain embodiments, AACM 115 selects a stratified group of users based on one or more similarities between the information in user profile 116 and user profiles of a pool of users in user database 110. For example, AACM 115 may use one or more similarity or stratification factors for the stratification, the one or more stratification factors including at least one of the user's disease progression info, medication info, demographic info, objectives, achievement of the corresponding objectives, interests, abilities, behavioral metrics, outcome metrics, or a combination thereof. Although, note that for a user who is just starting to use application 106, no behavioral and outcome metrics may be available yet.

In certain embodiments, additional stratification factors are also possible. For example, stratification may also be performed based on the user's interaction with application 106 during the set-up process (e.g., during the objective setting process). For example, stratification may be performed based on the user's likelihood to focus on one or more of physical activity, overall interaction with application 106, etc. In one example, application 106 may categorize the user based on three categories of: in denial, passively open, and actively open. In denial users may be users who refuse to accept that that they have diabetes, do not want to hear about it, or do not want to act on it. Passively open users are users who accept that they have diabetes, are open to information, and yet they may not be serious about taking all the steps and measures necessary to improve their health. Actively open users are users who accept they have diabetes, are eager to obtain more information, and are willing to be diligent about doing what it takes to improve their health. In certain embodiments, application 106 may categorize the user into one of these categories based on application 106's interaction with the user during set-up process (e.g., by asking questions that are designed to determine what category the user belongs to).

Note that, in certain embodiments, achievement of an objective may be used as a stratification factor. In certain embodiments, how a user has performed with respect to achieving a certain objective is indicated by a corresponding programmatic outcome metric that is calculated and stored in the user's profile for that certain objective. In certain embodiments, the achievement of an objective may be defined by a threshold. For example, a programmatic outcome metric of 100% with respect to a certain objective may indicate that a user in the pool of users has completely achieved the objective while a programmatic outcome metric of 90% may indicate that the user has progressed positively towards achieving the objective by accomplishing at least 90% of the objective (i.e., a loss of 9 pounds in weight instead of 10). With respect to stratification, in embodiments where achievement of an objective is used as a stratification factor, a threshold programmatic outcome metric may be defined. For example, the stratification factor may be a programmatic outcome metric of at least, e.g., 70%. In that case, users who have at least progressed towards achieving the objective by 70% will be included in the stratified group of users.

Examples of information about the user that may be used for selecting a stratified group includes 1) a 62 year-old woman on basal insulin only with three years of diabetes history, 2) 55 year-old man on basal and fast acting bolus therapy, 3) 70 year-old man on Metformin with heart disease, and 4) 40 year-old woman who is actively managing her di1abetes with diet and exercise.

One of a variety of methods and approaches may be used for stratifying user database 110 based on one or more stratification factors (e.g., disease progression, medication info, demographic information, objectives, achievement of the corresponding objectives, interests, abilities, behavioral metrics, outcome metrics, or a combination thereof). In certain embodiments, AACM 115 may use one of a variety of data filtering techniques to filter the broader user database 110 based on one or more stratification factors. For instance, if the user has Type I diabetes, AACM 115 may filter all user profiles in user database 110 who also have Type I diabetes. In that case, the stratified group of user would include all such users. In certain embodiments, however, if additional stratification factors are used for stratification, then additional filtering may be performed to further narrow the group of users in the stratified group. For example, if the stratification factors include disease progression and demographic info and the user is a male who has Type I diabetes, then AACM 115 may filter all user profiles of all male users in user database 110 who also have Type I diabetes.

In certain embodiments, AACM 115 may use machine learning algorithms to stratify user database 110. For instance, an unsupervised learning algorithm may be used for clustering all user profiles in user database 110 and determining to which cluster user profile 116 belongs. Unsupervised learning is a type of machine learning algorithm used to draw inferences from datasets consisting of input data without labeled responses. As one of ordinary skill in the art appreciates, in addition to an unsupervised learning algorithm that focuses on a clustering analysis, other types of unsupervised learning algorithms may be used.

In certain embodiments, a supervised learning algorithm may be used instead. Supervised learning is the machine learning task of learning a function that maps an input to an output based on example input-output pairs. In certain embodiments, using a supervised learning algorithm, AACM 115 may be configured to classify user profile 116 by determining what class or stratified group of users the user belongs to, based on a machine learning model that has been trained using a labeled dataset. In certain embodiments, the labeled data already includes different classes of users that are classified based on one or more characteristics, such as disease progression. For instance, in certain embodiments, one class of users includes all user profiles with Type I diabetes, while another class of users includes all user profiles with Type II diabetes. In such an example, if disease progression info 120 of user profile 116 indicates that the user has Type 1 diabetes, then AACM 115 selects that class as a stratified group of users for the user.

At step 306, operations 300 continue by configuring (e.g., automatically) the application based on information associated with the user, including the user's objectives, and/or information relating to the stratified group of users. Automatically configuring an application refers to configuring the application without user action or involvement. For example, an application may be automatically configured with a certain number of FFSCs that are selected by AACM 115 from a larger number of FFSCs that are available in the application binary file that is downloaded on user device 107.

Step 306 may be performed by AACM 115, in some embodiments. For example, AACM 115 may configure application 106 with application configuration 108 based on user profile 116 and/or user profiles of the stratified group of users. In certain embodiments, AACM 115 may analyze which one or more FFSCs have the strongest correlation with achieving the user's objectives 132 within the stratified group. In certain embodiments, AACM 115 then configures application 106 with at least a subset of such FFSCs, resulting in application configuration 108. In certain embodiments, the subset may include FFSCs with correlation scores above a certain correlation threshold. As described above, a FFSC refers to a certain combination of a feature and feature setting, such as, an exercise management feature 2 with setting 3. In certain embodiments, step 306 may be performed by application 106 itself. For example, AACM 115 may indicate the subset of FFSCs to application 106, which may then automatically configure itself with the subset of FFSCs, resulting in application configuration 108.

In one example, the user may be pre-diabetic and 77 years old. The user may also have the following objectives: lose 5 pounds and eat less carbs and more protein, etc. The user's interest may be hiking, walking, swimming, eating red meat, Italian food, and also a dislike for fish. In such an example, a stratified group of users may be selected based on the user's disease type and/or age. AACM 115 may then determine what FFSCs are most strongly correlated with losing 5 pounds and eating less carbs. For example, AACM 115 may determine that an exercise management feature, a reporting feature, a rewards feature, and a diet management feature, among others, are most strongly correlated with achieving the objectives above for users in the stratified group. That is, in such an example, profiles of users within the stratified group who have met those objectives may most frequently indicate the users' usage of one or more these FFSCs as well as high BEMs associated with such FFSCs. Note that in this example, achievement of those objectives are used as stratification factors.

One of ordinary skill in the art appreciates the different types of operations or algorithms that can be used for finding these correlations between achievement of the user's objectives and FFSCs, used by users in a stratified group, that are most strongly associated or correlated with achieving those objectives. Examples of algorithms that may be used include one or more of linear correlation algorithms (e.g., Pearson's Correlation Coefficient), non-linear correlation algorithms (e.g., Distance Correlation, Maximal Information Coefficient, etc.), as well as different types of machine learning algorithms.

In certain embodiments, a linear correlation algorithm may be used to determine the correlation between two variables, including a certain FFSC (first variable) with achieving a certain objective (second variable). In certain embodiments, the dataset based on which the correlation algorithm is executed includes data points from the user profiles of a number of users (e.g., users in the stratified group). The data points, in certain embodiments, include the FFSCs that each user utilizes, the user's objective(s), and whether the user has met those objectives. Using such a dataset, in some embodiments, AACM 115 may run a plurality of analyses, where each analysis focuses on the correlation between one of a possible number of FFSCs and one of the user's objective. Other types of analyses may be performed such that correlations between a user's objective and multiple FFSCs may be determined. As described above, instead of a correlation algorithm, in certain embodiments, a machine learning model may be used for the purpose of identifying features that are strongly correlated with achieving user's objectives. Different examples of algorithms or data models used for performing step 306 are described with respect to FIGs. 4A-5B.

Having determined a pool of FFSCs that are positively correlated with achieving the user's objectives, in certain embodiments, AACM 115 may then select all of those FFSCs to configure application 106 with, resulting in application configuration 108. In certain embodiments, AACM 115 may pick a subset of FFSCs from the pool of FFSCs that are very strongly correlated with achieving the user's objectives by applying, e.g., a correlation threshold. As an example, a pool of FFSCs may be initially determined, each FFSC having a certain correlation score, the correlation score indicating the correlation between the FFSC and achieving an objective of the user. AACM 115 may then select FFSCs having correlation scores above the threshold. Using the selected FFSCs, AACM 115 may the configure application 106, resulting in application configuration 108.

At step 308, operations 300 continue by evaluating behavioral metrics and/or outcome metrics of the user. In some embodiments, step 308 is performed by DAM 113. For example, after application 106 is configured with application configuration 108 and the user starts using application 106, DAM 113 may receive inputs 127 from application 106, based on which DAM 113 determines outcome metrics 130 and behavioral metrics 128. In some embodiments, outcome metrics 130 may be determined to evaluate whether the user is meeting their objectives or is at least improving. For example, a programmatic outcome metric may be calculated with respect to each of the user's objectives. The programmatic metric may indicate how close the user is to meeting their objective, using a percentage, for example.

As described above, in certain embodiments, behavioral metrics 128 include BEMs, where each BEM indicates the user's engagement with respect to a different FFSC. In certain embodiments, as described above, when calculating a BEM for a certain FFSC, DAM 113 takes into account other behavioral metrics that are relevant to the FFSC as well. BEMs, therefore, may be indicative of whether application configuration 108 needs to be adapted to be more effective in helping the user achieve their objectives.

In certain embodiments, as described above, outcome metrics 130 and behavioral metrics 128 are continuously calculated by DAM 113 in real-time and stored in user profile 116. The timing associated with all these metrics may also be stored (e.g., using time stamps), so trends can be calculated over time.

At step 310, operations 300 continue by adapting the application configuration based on information relating to at least some of the behavioral and/or outcome metrics of the user and/or information relating to at least some of the behavioral and/or outcome metrics of users (e.g., users in the stratified group) in the user database. Step 310 may be performed by AACM 115. For example, in certain embodiments, AACM 115 may adapt application configuration 108 based on information relating to users who exhibited similar behavioral metrics in connection with using all, or at least some, of the FFSCs of application configuration 108. Automatically reconfiguring an application refers to reconfiguring the application without user action or involvement. For example, an application may be automatically reconfigured by making certain FFSCs that the application 106 was previously configured with, unavailable, and also making certain new FFSCs available on application 106. In some embodiments, AACM 115 analyzes a dataset associated with the stratified group of users selected at step 304 to identify users who exhibited similar behavioral metrics in connection with using some or all of the FFSCs of application 106. In other words, AACM 115 further stratifies the already stratified group of users based on similarities in behavioral metrics in connection with using some or all of the FFSCs of application 106. This further stratification allows AACM 115 to predict what the user's behavior would be with respect to a FFSC that the user has not used yet. The reasoning is that if the user and users in this further stratified group showed similar behavior with respect to a first set of FFSCs, then the user is likely going to exhibit behavior that is similar to the behavior that the users in the further stratified group have exhibited towards a second set of FFSCs that the user has not used yet.

To illustrate this with an example, application configuration 108 may include exercise management feature 1 with setting 2, exercise management feature 3 with setting 1, sleep management feature 2 with setting 5, reporting feature 1 with setting 3, etc. The user's BEMs associated with such FFSCs may be, respectively, as follows: 20%, 90%, 68%, and 80%.

An analysis by AACM 115 may then show that users in the stratified group selected at step 304, who met their objective(s) and exhibited similar behavioral engagement metrics with respect to those exact FFSCs, showed above-90% BEMs with respect to exercise management feature 1 with setting 5. Accordingly, AACM 115 may determine to swap exercise management feature 1 with setting 2 with exercise management feature 1 with setting 5 but keep the rest of the FFSCs unchanged.

In certain embodiments, a determination of whether to keep or swap a certain FFSC may be dependent on whether the FFSC's BEM is below a certain threshold and/or whether the user is meeting their respective objective. In the example above, the user may have two objectives: lose 5 pounds and sleep uninterrupted at night. Based on the programmatic outcome metric of each objective, AACM 115 may determine that the user is not meeting the first objective but is meeting the second one. As such, because the BEM of exercise management feature 1 with setting 2 is below 70% (e.g., a configured threshold) and the respective objective of losing 5 pounds is not met, in certain embodiments, AACM 115 changes exercise management feature 1 with setting 2 to help the user meet their objective. However, in certain embodiments, although the behavioral engagement metric of sleep management feature 2 with setting 5 is below 70%, AACM 115 may determine to retain the FFSC because the user is meeting the objective. In some embodiments, AACM 115 may be configured to replace a FFSC with a BEM below the configured threshold, even if the user is meeting a corresponding objective. For example, AACM 115 may be configured to replace sleep management feature 2 with setting 5 with a BEM of below 70% even though the user is meeting their sleep-related objective.

In certain embodiments, steps 308 and 310 are performed frequently as real-time or more recent information (e.g., behavioral metrics 128 and outcome metrics 130) is obtained or generated about the user. As a result, in certain embodiments, AACM 15 frequently reconfigures application 106 with FFCSs that are more likely to engage the user and, thereby, help the user achieve their objectives. In certain embodiments, if the user changes their objectives, AACM 115 may perform at least some of the steps of operations 300 again. For example, AACM 115 may perform steps 304 and 306 again to replace some of the user's existing FFCSs, which related to the objective the user is no longer interested in, with new FFSCs that are associated with the user's new objective. In certain embodiments, which FFSCs are associated with the user's new objective may be determined in step 306, where AACM 15 identifies FFCSs that are highly correlated with the achievement of the user's objective by evaluating information associated with users in a stratified user group who were able to achieve the same objective or at least had the same objective.

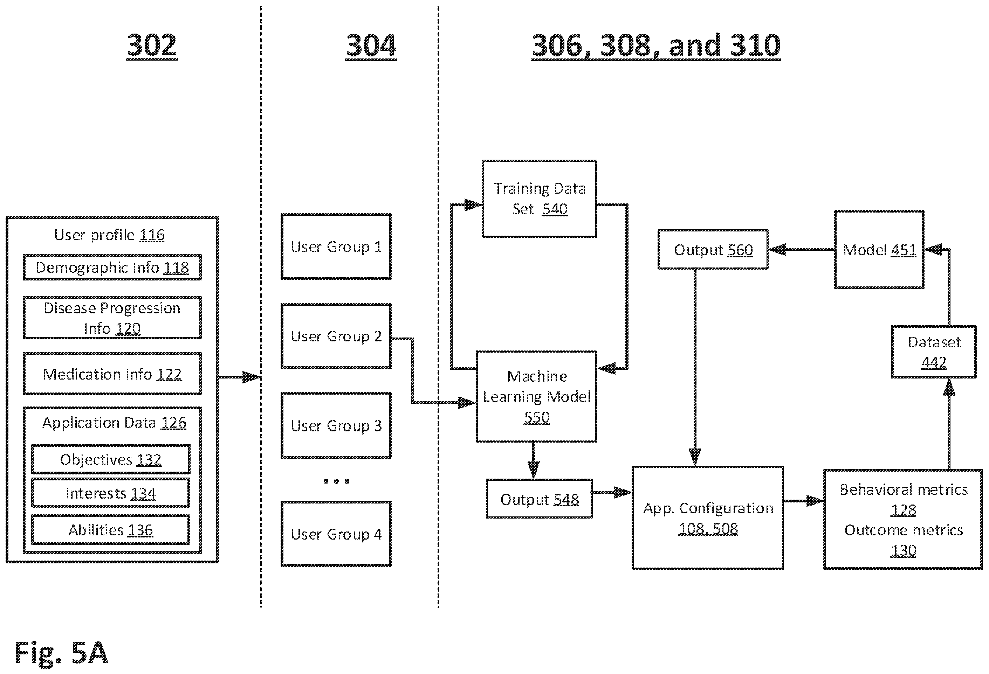

In certain embodiments, one of a variety of data models may be used to perform steps 302-310 of operations 300. One example data model is described in relation to FIGs. 4A-4B. A second example data model is described in relation to FIGs. 5A-5B. As one of ordinary skill in the art appreciates, there are a variety of approaches for use in adapting application configuration 108 to better help the user in meeting their objectives and avoiding plateau and boredom in the user's behavior towards application 106, thereby reducing the application's attrition rates. As such, the data models described in relation to FIGs. 4A-5B are merely exemplary.

FIG. 4A is a diagram illustrative of how application configuration 108 is initially selected and then adapted using a data model, which includes algorithm 450 and model 451, in accordance with certain embodiments. FIG. 4A is described with reference to FIG. 4B, which illustrates a number of datasets used in the operation of the data model of FIG. 4A. As described above, during step 302 of operation 300 in FIG. 3, objectives 132, interests 134, and/or abilities 136 of the user are identified and stored in user profile 116, which already also includes the user's demographic info 118, disease progression 120, and medication info 122. In the example of FIG. 4A, at this stage, no information about the user's behavioral and outcome metrics have yet been obtained because the user is a new user, who has not yet used the features of application 106. User profile 116 also does not comprise any application configuration information for the same reason. That is, application 106 has not been configured yet at this point for any application configuration information to be available. As a result, in the example of FIG. 4A, all the user-specific information that may be available at step 302 of operations 300 is the user's demographic info 118, disease progression info 120, medication info 122, objectives 132, interest 134, and/or abilities 136.

Using the available information, by performing step 304 of operations 300, AACM 115 stratifies user database 110, which comprises a pool of user profiles, to identify a stratified group of users that are similar to the user in certain aspects. As one of ordinary skill in the art appreciates, one of a variety of data filtering techniques or algorithms may be used when stratifying user database 110. In the example of FIG. 4A, AACM 115 stratifies user database 110 based on a set of stratification factors including the user's demographic info 118, disease progression info 120, medication info 122, objectives 132, interests 134, and/or abilities 136. For example, the user may be 77 year old male, with Type I diabetes, who takes insulin shots. The user's objective is to lose 5 pounds and his interests include running, swimming, and biking, but he is not able to exercise during weekends. Note that the user may have more than one objective but, for simplicity, algorithms 450 and 451 are described herein with respect to one objective.

Having stratified user database 110, AACM 115 identifies user group 2 as the stratified group of users who are all male, within the age of 67-87, with Type I diabetes, who take insulin shots. The users in user group 2 also all had a goal of losing weight and have met that goal. In other words, in the example of FIGs. 4A-4B, users in user group 2 were not only selected based on having the same objective as the user but also based on whether they met the objective. Whether a user has been able to meet their goal, as described above, may be indicated by a programmatic outcome metric associated with the user's objective. As such, in this example, achievement of the user's objective is used as a stratification factor. As discussed, achieving an objective may be defined in terms of a threshold programmatic outcome metric. In such an example, the threshold programmatic outcome metric may be used as the stratification factor such that all users with programmatic outcome metrics of less than a certain threshold (e.g., 70%, 80%, 100%, etc.) would then be dropped from the stratified group of users. AACM 115 may also take the user's interests 134 and abilities 136 into account when stratifying user database 110. For example, the users of user group 2 may all have the same interests and abilities as the user.

In some embodiments, when selecting a stratified group of users, AACM 115 may be configured to define a range around each of the stratification factors. For example, AACM 115 may be configured to define a broad user group 2 by including all users whose objectives were to lose weight (any amount) and were able to meet that objective. In some other examples, AACM 115 may be configured to define a narrower user group 2 by including only those user whose objective was to lose an amount of weight within a certain range of 5 pounds (e.g., any amount between the range of 4-6 pounds, or 3-7 pounds) or even exactly 5 pounds and were able to meet that objective. With respect to the other stratification factors, for example, although the user is 77 years old, in certain embodiments, user group 2 may include all users in the age range of 67-87. In the case of interests and abilities, as an example, in certain embodiments, AACM 115 may also include users whose interests are walking and weight lifting and users who have some time limitations with respect to when they can or cannot engage in exercising.

In some embodiments, the stratification may be performed by filtering the dataset available for all users in user database 110 based on one or more of the stratification factors discussed above. An example of a dataset including information about all users in user database 110 is shown as dataset 480 in FIG. 4B. As shown, each row in dataset 480 belongs to a different user while each column corresponds to a different data point provided by the user's profile. For example, the columns include the user's objectives, demographic info, disease progression, medicine info, interests, abilities, and FFSCs. Note that although a single column is used for the user's objectives, interests, and abilities, each of those columns may be representative of a plurality of columns. For example, dataset 480 may comprise a plurality of objective-related columns, each objective-related column corresponding to a certain possible objective that the user may have selected. In the example of FIG. 4B, the value recorded for each objective corresponds to the programmatic outcome metric associated with that objective, which indicates whether the user has met that objective or not. A "1" indicates that the user has met that objective while a "0" indicates the opposite. For example, dataset 480 shows that User 1 has met their objective. If no value is recorded for a certain objective, it means that the user has not selected that objective for themselves.

Note that, as discussed, programmatic outcome metrics may also be defined in terms of percentages. As a result, in certain embodiments, percentages may be recorded in the dataset for indicating programmatic outcome metrics. In certain other embodiments, zeroes and ones may still be used for simplicity by using threshold programmatic outcome metrics. For example, in certain embodiments, a "1" may be recorded in the dataset for any user with a programmatic outcome metric of more than a certain percentage (e.g., 70%). Dataset 480 also includes a set of FFSCs. For each FFSC, the BEM associated with the FFSC is recorded. For example, for FFSC 1 of User 1, a BEM of 90% is recorded, which shows that User 1 is very engaged with respect to FFSC 1. As shown, no value is recorded for FFSC 2 with respect to User 1, indicating that User 1 has not used that FFSC yet.

FIG. 4B also illustrates dataset 440 which is the result of stratifying dataset 480 based on the stratification factors described above. For example, User 2 and User 3 were eliminated from dataset 440 because User 2 was not able to meet their objective while user 3 did not even have the same objective as the user (i.e., user 102),

In certain embodiments, once dataset 440 is defined, AACM 115 then uses algorithm 450 that takes at least part of dataset 440 as input and outputs a ranked list of FFSCs based on their correlations to the achievement of the user's objective. How strongly a FFSC is correlated to the achievement of the user's objective may be determined based on a number of factors including the number of users in user group 2 who used the FFSC as well as the level of engagement (e.g., the corresponding BEMs) of such users with respect to the FFSC. As an example, the larger the number of users who used a certain FFSC and/or the higher the level of engagement towards the FFSC, the higher the likelihood that the user (i.e., user 102) would be engaged by that FFSC and, thereby, meet their objective. In one simplified example, the correlation (C) of each FFSC to the achievement of the user's objective may be defined as:

$$C = (W_1)*(N) + (W_2)*(\text{Average BEM})$$

In the formula above, $W_1$ is a defined weight, N is the number of users using the FFSC, $W_2$ is another defined weight, and the Average BEM is the average of the BEMs of all the users in dataset 440 for the corresponding FFSC. Using the formula above, $C_1$ through $C_n$ is calculated for FFSC 1 through FFSC N in dataset 440. $C_1$ through $C_n$ are then ranked in order from high to low so that FFSCs with the highest correlations can be identified. Algorithm 450, therefore, outputs a list of FFSCs, ranked based on correlations to the achievement of the user's objective, which is shown as output 460 in FIG. 4A. AACM 115 may then select a number of FFSCs from the list to include in application configuration 108. For example, AACM 115 may select the top ten, or any other defined number of, FFSCs from the list.

Note that although in the example provided with respect to FIGs. 4A and 4B achievement of the user's objective was used as a stratification factor, in certain other embodiments, such an approach it not necessary. For example, in certain embodiments, stratification may be performed not based on the achievement of the objective but based on the user's objective such that the stratified group of users would include users who all had the same objective as the one selected by the user. In such embodiments, the stratified group of users would include users who performed differently with respect to that objective. In such embodiments, as one of ordinary skill in the art appreciates, correlation algorithm(s) may be used to still find the FFSCs with the highest correlation to the achievement of the objective, which may be defined using a threshold programmatic outcome, as described above. In yet certain other embodiments, stratification may be performed based on factors unrelated to the user's objective, such as the user's demographic information, disease progression, etc. Similarly, in such embodiments, the stratified group of users would include users who did not even have the same objective as the user. In such embodiments, as one of ordinary skill in the art appreciates, correlation algorithm(s) may be used to still find the FFSCs with the highest correlation to the achievement of the objective, which may be defined using a threshold programmatic outcome, as described above.

Once application 106 is configured with application configuration 108, the user begins to use the selected FFSCs. Over time, as described above, application 106 receives inputs 127, including inputs 210 and 220, which DAM 113 uses to calculate behavioral metrics 128 and outcome metrics 130. As part of behavioral metrics 128, a BEM is calculated for each of the FFSCs of application configuration 108. Based on the user's BEMs, AACM 115 is able to further adapt application configuration 108 using model 451 (can also be referred to as model 451). For example, AACM 115 may create a dataset 442 including the user's BEM data 490 as well as BEM data 441, which is part of dataset 440 associated with the users in user group 2. In certain embodiments, by evaluating similarities between the user's BEM data 490 and other users' BEM data 441, model 451 is able to recommend FFSCs that are most likely going to engage the user and thereby, help them meet their objective and improve their health.

In the example of FIG. 4A, model 451 is a recommender model (e.g., a type of machine learning model) that uses memory-based, user-user (read as "user to user" or also referred to as "user to item") collaborative filtering, which is configured to recommend items (FFSCs in this example) to a user based on similarities between the user's behavior and the behaviors of a pool of users in a dataset. For example, based on similarities between the user's BEM data 490 and other users' BEM data in dataset 441, model 451 may recommend one or more FFSCs that the user has not yet used, which are likely to help the user achieve their objective. A memory-based user-user collaborative filtering algorithm is built on the premise that users who behave the same way towards an item (e.g., FFSC 1) will also behave similarly towards another item (e.g., FFSC 3).

As an example, in dataset 442, the user has a 93% BEM for FFSC 1, an 18% BEM for FFSC 2, and a 25% BEM for FFSC N. In this example, model 451 may determine that the user behaves more similarly to users 1 and 4 as opposed to user 8. Similarly, in certain embodiments, model 451 may identify other users who have acted similarly with respect to the same FFSCs used by user 102 and then determine a set of FFSCs that are highly likely to engage the user and thereby help them achieve their objective. This determined set of FFSCs is shown as output 455 in FIG. 4A. In certain embodiments, AACM 115 then utilizes this new set of FFSCs to reconfigure application configuration 108, resulting in application configuration 408. For example, AACM 115 may remove some of the FFSCs, with respect to which user 102 did not show much engagement, replace them with some of the FFSCs from output 445, etc.

By adapting application configuration 108 to replace one or more FFSCs that were not helpful to the user with one or more FFSCs of this new set of FFSCs, AACM 115 is able to increase the likelihood of the user achieving their objective.

The training of model 451 may be performed by AACM 115 or one or more processors or computing systems in data communication with decision support engine 112. Model 451 may be a new model initialized with random weights and parameters, or may be partially or fully pre-trained (e.g., based on prior training rounds). Model 451 may be trained using algorithms such as message-passing algorithms. Model 451 may be fine-tuned or continue to be retrained as model 451 continues to provide output 445 (e.g., feature recommendations) and as dataset 442 is updated with the specific user's behavioral metrics 128 and outcome metrics 130 corresponding to the output 445. Note that although in the example of FIG. 4A data filtering is used to obtain dataset 440 belonging to the stratified user group 2, one of a variety of other techniques may be used instead. For example, in some embodiments, an unsupervised machine learning algorithm may be used to determine which cluster of users the user belongs to. For example, an unsupervised machine learning algorithm may be trained over a period of time by a dataset associated with the entire pool of users in user database 110. The trained unsupervised machine learning algorithm may then be configured to cluster the user by taking as input a vector for the user that comprises information relating to all the stratification factors above. As a result of this exercise, a cluster of users (e.g., user group 2), is identified and a dataset (e.g., dataset 440) comprising information relating to the users in that cluster is obtained. As one of ordinary skill in the art appreciates, other types of algorithms may also be used for selecting a stratified group of users and obtaining a corresponding dataset.

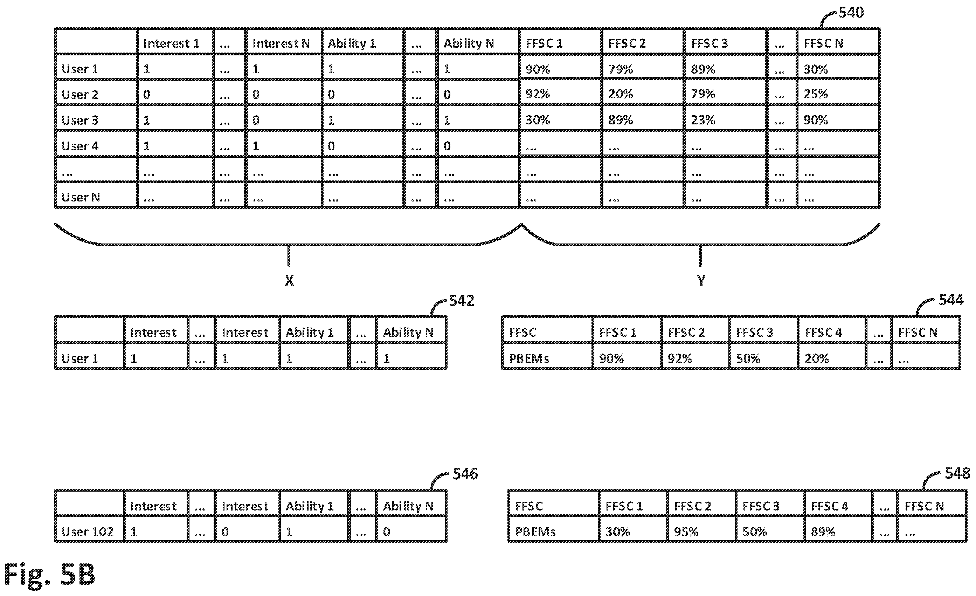

FIG. 5A is a diagram illustrative of how application configuration 108 is initially selected and then adapted using a data model including a machine learning model 550 and model 451. FIG. 5A is described with reference to FIG. 5B, which illustrates a number of datasets used in the operations of the data model of FIG. 5A. As described above, during step 302 of FIG. 3, objectives 132, interests 134, and/or abilities 136 of the user are identified and stored in user profile 116, which already also includes the user's demographic info 118, disease progression 120, and medication info 122. Similar to FIG. 4A, in the example of FIG. 5A, all the user-specific information that may be available at this stage is the user's demographic info 118, disease progression info 120, medication info 122, objectives 132, interest 134, and/or abilities 136.

In the embodiments of FIG. 5A, by performing step 304 of operations 300, AACM 115 stratifies user database 110 based on the user's demographic info 118, disease progression info 120, and/or medication info 122. As described above, one of a variety of techniques may be used for this stratification. What results from the stratification is, for example, user group 2, which includes a pool of users who are similar to the user with respect to their demographic info, disease progression info, and medication info.

In certain embodiments, having identified user group 2 as the stratified group, in order to determine an initial set of FFSCs for application configuration 108, AACM 115 uses information associated with the user as input to a machine learning model 550 that is trained specifically for user group 2. In certain embodiments, machine learning model 550 may be trained using one of a variety of datasets. FIG. 5B illustrates a dataset 540 of all users in user group 2 who chose the same objective as the user and have also met that objective. Dataset 540 indicates each user's interests and abilities as well as their BEMs associated with FFSCs that they have interacted with. Machine learning model 550 (i.e., F(X)) is trained by providing as input information about each user's interests and abilities (e.g., the portion of each row, referred to as X) and obtaining as output a predicted set of BEMs ("PBEMs") for FFSCs 1-N (e.g., Y'). The predicted set of BEMs (e.g., Y') may then be compared with the user's actual set of BEMs (i.e., the portion of each row, referred to as Y) to determine machine learning model 550's error. Machine learning model 550 may then be adjusted using a training algorithm based on the error in order to configure the model to predict with more accuracy. The training of machine learning model 550 may be performed by AACM 115 or one or more processors or computing systems in data communication with decision support engine 112.

For example, during the training process, vector 542, which includes information about User 1's interests and abilities, is fed into machine learning model 550 as "X." Machine learning model 550 then outputs a vector 544 as "Y'." User 1's actual BEMs (i.e., "Y"), shown in dataset 540, are then compared with vector 544 to determine the error, based on which machine learning model 550 is trained.

Similarly, for the user (i.e., user 102), a vector 546 may be fed into machine learning model 550, which then outputs a vector 548 including a set of PBEMSs corresponding to FFSCs 1-N. In certain embodiments, AACM 115 may then select a subset of FFSCs 1-N in vector 548 based on the PBEMs. For example, AACM 115 may select FFSC's with the highest PBEMs in each category. As an example, if there are ten different FFSCs (e.g., FFSC 1-10) in vector 548 related to exercise management, AACM 115 may select the two with the highest PBEMs. In certain embodiments, AACM 115 then configures application 106 with application configuration 108, which includes the selected subset of FFSCs.

Note that in the example of FIG. 5B, the input to machine learning model 550 only includes information about the user's interests and abilities because the dataset has already been stratified for other types of information, such as demographic information, disease progression, medicine information, objective, achievement of the objective, etc. However, in other examples, additional inputs may be included in a dataset that is used to train machine learning model 550. Similarly, such additional inputs may be included when information associated with user 102 is fed into the model 550 to obtain a set of PBEMs.

Once application 106 is configured with application configuration 108, the user starts using the corresponding FFSCs over time, during which behavioral metrics 128 and outcome metrics 130 are obtained. As part of behavioral metrics 128, a BEM is calculated for each of the FFSCs of application configuration 108. At this stage, AACM 115 is in possession of information relating to user's behavior, which AACM 115 is able to use to further adapt application configuration 108. For example, as was described above with respect to FIG. 4B, AACM 115 may create a dataset 442 that AACM 115 may use as input into a recommender model 451. Recommender model 451 then generates output 560, which corresponds to a set of FFSC's that are highly likely to engage the user and thereby help user achieve their objective. AACM 115 then utilizes this new set of FFSCs to reconfigure application configuration 108, resulting in application configuration 508, as described above.

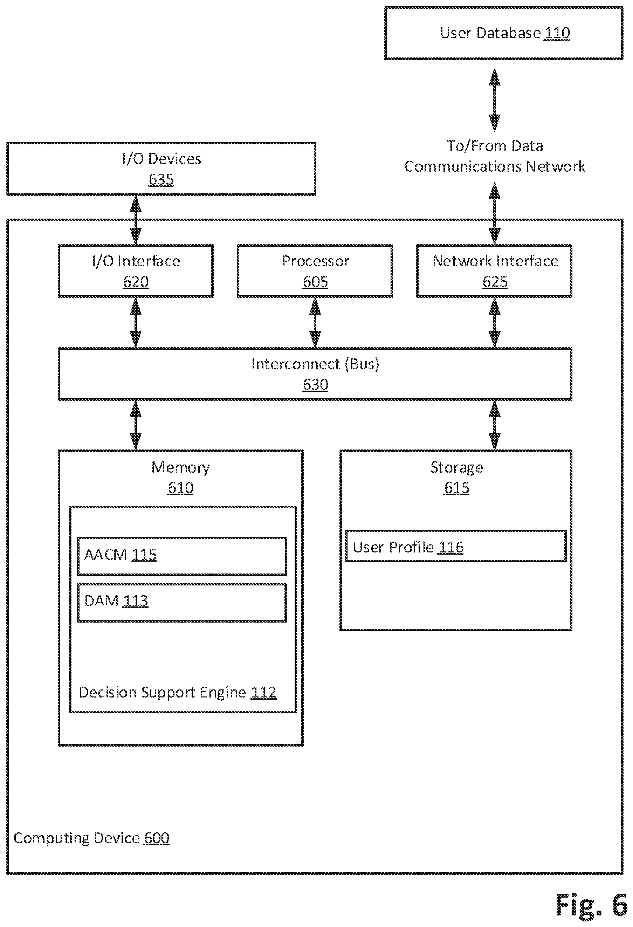

FIG. 6 is a block diagram depicting a computing device 600 configured to select and continuously adapt an application configuration of an application executed by either computing device 600 or another computing device in communication with computing device 600, according to certain embodiments disclosed herein. Although depicted as a single physical device, in embodiments, computing device 600 may be implemented using virtual device(s), and/or across a number of devices, such as in a cloud environment. As illustrated, computing device 600 includes a processor 605, memory 610, storage 615, a network interface 625, and one or more I/O interfaces 620. In the illustrated embodiment, processor 605 retrieves and executes programming instructions stored in memory 610, as well as stores and retrieves application data residing in storage 615. Processor 605 is generally representative of a single CPU and/or GPU, multiple CPUs and/or GPUs, a single CPU and/or GPU having multiple processing cores, and the like. Memory 610 is generally included to be representative of a random access memory. Storage 615 may be any combination of disk drives, flash-based storage devices, and the like, and may include fixed and/or removable storage devices, such as fixed disk drives, removable memory cards, caches, optical storage, network attached storage (NAS), or storage area networks (SAN).

In some embodiments, input and output (I/O) devices 635 (such as keyboards, monitors, etc.) can be connected via the I/O interface(s) 620. Further, via network interface 625, computing device 600 can be communicatively coupled with one or more other devices and components, such as user database 110. In certain embodiments, computing device 600 is communicatively coupled with other devices via a network, which may include the Internet, local network(s), and the like. The network may include wired connections, wireless connections, or a combination of wired and wireless connections. As illustrated, processor 605, memory 610, storage 615, network interface(s) 625, and I/O interface(s) 620 are communicatively coupled by one or more interconnects 630. In certain embodiments, computing device 600 is representative of mobile device 107 associated with the user. In certain embodiments, as discussed above, the mobile device 107 can include the user's laptop, computer, smartphone, and the like. In another embodiment, computing device 600 is a server executing in a cloud environment.

In the illustrated embodiment, storage 615 includes user profile 116. Memory 610 includes decision support engine 112, which itself includes AACM 115 and DAM 113. Decision support engine 112 is executed by computing device 600 to perform operation 304-310 of operations 300 in FIG. 3. AACM 115 is configured with or comprises any algorithms (e.g., algorithms 450 and 451) necessary for the operations of data models described in relation to FIGs. 4A-5B.

Accordingly, certain embodiments described herein provide a technical solution to a technical problem in the technical field of personalization or adaptation of the configuration of a diabetes or health-related software application (e.g., mobile application) in order to assist the user in improving their health or managing a disease. Automatically reconfiguring an application by changing the set of features and feature settings (e.g., making some available and others unavailable) based on the user's own information and information associated with a certain set of users who are similar to the user in one or more aspects is a technical improvement to how (1) a health-related software application operates and (2) how a health monitoring system, including a glucose monitoring system and a health-related software application, operates. For example, as described above, in certain embodiments, an application configuration with a set of FFSCs may be initially selected for the user based on the user's own information (e.g., objectives, interests, abilities, demographic information, disease progression, medication info, etc.) and/or information associated with a certain set of users who are similar to the user in one or more aspects. In certain embodiments, this certain set of users are users who have/had the same objectives as the user and/or met the objectives. In certain embodiments, information associated with this certain set of users includes the users' behavioral information associated with FFSCs used by the users and/or whether the users were able to achieve the same objectives. In addition, in certain embodiments, the initial application configuration may be frequently reconfigured based on (1) the user's own behavioral information as well as information associated with the user's health and/or performance towards the user's objectives and (2) users who showed similar behavior towards those same objectives and/or health-related performance towards the same objectives.

In certain embodiments, using the information described above to configure the application with an initial configuration and frequently reconfiguring the application based on the additional information described above as well as the techniques described herein improves the technical field of personalization or adaptation of the configuration of a diabetes or health-related software application. An application configured based on the embodiments described herein is more likely to engage the user and, thereby, help the user improve their health. Note that with respect to a health-related, and especially a disease management application, personalizing an application based on the information and techniques/algorithms described herein may make a significant difference in the user's life and health, to the extent where the application's guidance may in some cases help save the user's life. Accordingly, the difference between an application that is not personalized or personalized ineffectively and an application that is personalized based on the embodiments described herein may be the difference between a user who is not engaged and stops using the application, thereby possibly resulting in a deterioration of the user's health, and a user who is engaged with the application and is able to effectively manage their disease and improve their health through the personalized guidance and features provided to the user.

Each of these non-limiting examples can stand on its own or can be combined in various permutations or combinations with one or more of the other examples The above detailed description includes references to the accompanying drawings, which form a part of the detailed description The drawings show, by way of illustration, specific embodiments in which the invention can be practiced. These embodiments are also referred to herein as ▪ 'examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, tire term "or" is used to refer to a nonexclusive or, such that "A or B" includes "A but not B," "B but not A," and "A and B," unless otherwise indicated. In this document, the terms "including" and "in which" are used as the plain English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Geometric terms, such as "parallel", "perpendicular", "'round", or "square", are not intended to require absolute mathematical precision, unless the context indicates otherwise. Instead, such geometric terms allow for variations due to manufacturing or equivalent functions. For example, if an element is described as "round" or "generally round", a component that is not precisely circular (e.g., one that is slightly oblong or is a many-sided polygon) is still encompassed by this description.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment.

**Claims**

**1.** A system comprising:

a memory circuit; and
a processor configured to:

receive a request to configure an application for use by a user, wherein the application is at least partially resident on a computing device to manage sensor data generated by a glucose monitoring system associated with the user;
identify an objective for the user;
identify classifying information associated with the user, the classifying information including at least one of the objective, interest, ability, demographic information, disease progression information, or medication regimen information of the user;

select a group of users from among a pool of users based on one or more similarities between the user and the group of users with respect to the identified classifying information;

identify one or more application features from a plurality of application features based on the objective of the user and a correlation of each of the plurality of application features with the objective in a dataset associated with the group of users; and

automatically configure the application with the one or more application featureswherein the processor is further configured to:

receive a plurality of inputs including:

a first input including glucose measurements associated with the user generated by the glucose monitoring system; and

a second input indicative of behavior of the user with respect to the one or more application features;

calculate a programmatic outcome metric associated with the objective based at least on the first input, wherein the programmatic outcome metric is indicative of an extent to which the user has achieved the objective;

calculate, based on the second input, one or more behavioral engagement metrics (BEMs) for the one or more application features, such that a separate BEM is calculated for each of the one or more application features;

identify one or more users in the selected group of users or the pool of users with BEMs similar to the calculated one or more BEMs;

identify a new application feature not included in the one or more features based on the feature being associated with a BEM above a threshold for at least one of the one or more users; and

reconfigure the application with the new application feature based on at least one of the one or more BEMs and the programmatic outcome metric.

2. The system of claim 1, wherein the processor being configured to identify the objective comprises the processor being configured to:

receive user input relating to what the user intends to achieve with respect to the user's diabetes; and

convert the user input into the objective based on one or more defined guidelines, and optionally, wherein the processor being configured to convert the user input into the objective based on the one or more defined guidelines comprises the processor being configured to:

categorize the user into a category based on the guidelines and information associated with the user, wherein:

the information associated with the user includes the classifying information, and the guidelines indicate the objective for the category; and

select the objective for the user based on the categorization.

3. The system of claim 1, wherein the processor being configured to identify the objective comprises the processor being configured to:

receive user input relating to what the user intends to achieve with respect to the user's diabetes; and

convert the user input into the objective based on information associated with the group of users.

4. The system of claim 1, wherein the processor being configured to reconfigure the application with the new application feature comprises the processor being configured to:

identify a low performing application feature of the one or more application features with a corresponding BEM that is below a threshold; and

replace the low performing application feature of the one or more application features with the new application feature, and optionally, wherein the processor being configured to reconfigure the application with the new application feature comprises the processor being configured to:

identify that the low performing application feature of the one or more application features relates to the objective; and

identify that the programmatic outcome metric is below a threshold.

5. A method of configuring an application with one or more application features, comprising:

receiving a request to configure the application for use by a user, wherein the application is at least partially resident on a computing device to manage sensor data generated by a glucose monitoring system associated with the user;

identifying an objective for the user;

identifying classifying information associated with the user, the classifying information including at least one of the objective, interest, ability, demographic information, disease progression information, or medication regimen information of the user;

selecting a group of users from among a pool of users based on one or more similarities between the user and the group of users with respect to the identified classifying information; identifying the one or more application features from a plurality of application features based on the objective of the user and a correlation of each of the plurality of application features with the objective in a dataset associated with the group of users;

configuring the application with the one or more application features;

receiving a plurality of inputs including:

a first input including glucose measurements associated with the user generated by the glucose monitoring system; and

a second input indicative of behavior of the user with respect to the one or more application features;

calculating a programmatic outcome metric associated with the objective based at least on the first input, wherein the programmatic outcome metric is indicative of an extent to which the user has achieved the objective;

calculating, based on the second input, one or more behavioral engagement metrics (BEMs) for the one or more application features, such that a separate BEM is calculated for each of the one or more application features;

identifying one or more users in the selected group of users or the pool of users with BEMs similar to the calculated one or more BEMs;

identifying a new application feature not included in the one or more features based on the feature being associated with a BEM above a threshold for at least one of the one or more users; and

reconfiguring the application with the new application feature based on at least one of the one or more BEMs and the programmatic outcome metric.

6. The method of claim 5, wherein identifying the objective further comprises:

receiving user input relating to what the user intends to achieve with respect to the user's diabetes; and

converting the user input into the objective based on one or more defined guidelines, and optionally, wherein the converting further comprises:

categorizing the user into a category based on the guidelines and information associated with the user, wherein:

the information associated with the user includes the classifying information, and the guidelines indicate the objective for the category; and

selecting the objective for the user based on the categorization.

7. The method of claim 5, wherein identifying the objective comprises:

receiving user input relating to what the user intends to achieve with respect to the user's diabetes; and

converting the user input into the objective based on information associated with the group of users.

8. The system of claim 3 or method of claim 7, wherein the information associated with the group of users includes one or more glucose-related metrics of the group of users.

9. The system of claim 1 or method of claim 5, wherein selecting the group of users is further based on a programmatic outcome metric of each of the pool of users with respect to the objective, and optionally, wherein:

programmatic outcome metrics of the selected group of users are above a threshold programmatic outcome metric associated with achievement of the objective, and

the threshold programmatic outcome metric associated with achievement of the objective is indicative of a defined minimum amount of positive progression towards achieving the objective.

10. The system of claim 1 or method of claim 5, wherein the correlation of each of the plurality of application features with the objective comprises a correlation of each of the plurality of application features with achievement of the objective, or, wherein the correlation of each of the plurality of application features with the objective is based on a number of users in the selected group of users who used the feature and behavioral engagement of the number of users with respect to the feature, and optionally, wherein behavioral engagement of each of the number of users with respect to the application feature is indicated by a behavioral engagement metric (BEM) of each user of the number of users with respect to the application feature, and wherein the BEM is based on an interaction of each user of the number of users with the feature, the interaction including at least one of:

> a frequency with which each user of the number of users interacts with the application feature;
> a frequency with which each user of the number of users ignores a guidance generated by the application feature;
> an average amount of time each user of the number of users spends interacting with the application feature; or
> how closely behavior of each user of the number of users adheres to the guidance generated by the application feature.

11. The system of claim 4 or method of claim 5, wherein each BEM of the one or more BEMs is based on an interaction of the user with a corresponding application feature of the one or more application features, the interaction including at least one of:

> a frequency with which the user interacts with the corresponding application feature;
> a frequency with which the user ignores a guidance generated by the corresponding application feature;
> an average amount of time the user spends interacting with the corresponding application feature; or
> how closely behavior of the user adheres to the guidance generated by the corresponding application feature.

12. The method of claim 11, wherein reconfiguring the application with the new application feature comprises:

> identifying a low performing application feature of the one or more application features with a corresponding BEM that is below a threshold; and
> replacing the low performing application feature of the one or more application features with the new application feature, and optionally, wherein reconfiguring the application with the new application feature comprises:

>> identifying that the low performing application feature of the one or more application features relates to the objective; and
>> identifying that the programmatic outcome metric is below a threshold.

13. The system of claim 1 or method of claim 5, wherein each of the one or more application features has a correlation with the objective that is above a correlation threshold, or, wherein each application feature of the one or more application features comprises a feature setting.

14. A non-transitory computer readable medium having instructions stored thereon that, when executed by a processor, causes a computing system to perform the method of any of claims 5 to 13.

**Patentansprüche**

1. System, umfassend:

> eine Speicherschaltung; und
> einen Prozessor, der konfiguriert ist zum:

>> Empfangen einer Anfrage zur Konfiguration einer Anwendung für die Verwendung durch einen Benutzer, wobei die Anwendung mindestens teilweise auf einer Rechenvorrichtung vorhanden ist, um Sensordaten zu verwalten, die von einem dem Benutzer zugeordneten Glukoseüberwachungssystem erzeugt werden;
>> Identifizieren eines Ziels für den Benutzer;
>> Identifizieren von Klassifizierungsinformationen, die dem Benutzer zugeordnet sind, wobei die Klassifizierungsinformationen mindestens eine der folgenden Informationen einschließen: Ziel, Interessen, Fähigkeiten, demografische Informationen, Krankheitsverlauf oder Medikamentenplan des Benutzers;
>> Auswählen einer Benutzergruppe aus einem Benutzerpool basierend auf einer oder mehreren Ähnlichkeiten zwischen dem Benutzer und der Benutzergruppe in Bezug auf die identifizierten Klassifizierungsinformationen;
Identifizieren eines oder mehrerer Anwendungsmerkmale aus einer Vielzahl von Anwendungsmerkmalen basierend auf dem Ziel des Benutzers und einer Korrelation jedes der Vielzahl von Anwendungsmerkmalen mit dem Ziel in einem der Benutzergruppe zugeordneten Datensatz; und

automatisches Konfigurieren der Anwendung mit der einen oder den mehreren Anwendungsmerkmalen, wobei der Prozessor ferner konfiguriert ist zum:
Empfangen einer Vielzahl von Eingaben, die Folgendes einschließen:

eine erste Eingabe, die vom Glukoseüberwachungssystem erzeugte und dem Benutzer zugeordnete Glukosemesswerte einschließt; und
eine zweite Eingabe, die das Verhalten des Benutzers in Bezug auf das eine oder die mehreren Anwendungsmerkmale anzeigt;
Berechnen einer programmatischen Ergebniskennzahl, die dem Ziel zugeordnet ist, basierend mindestens auf der ersten Eingabe, wobei die programmatische Ergebniskennzahl angibt, inwieweit der Benutzer das Ziel erreicht hat;
basierend auf der zweiten Eingabe, Berechnen einer oder mehrerer Verhaltensnutzungskennzahlen (BEM) für das eine oder die mehreren Anwendungsmerkmale, so dass für jedes des einen oder der mehreren Anwendungsmerkmale eine separate BEM berechnet wird;
Identifizieren eines oder mehrerer Benutzer in der ausgewählten Benutzergruppe oder dem Benutzerpool, deren BEMs der einen oder den mehreren berechneten BEMs ähnlich sind;
Identifizieren eines neuen Anwendungsmerkmals, das nicht in dem einen oder den mehreren Merkmalen enthalten ist, basierend darauf, dass das Merkmal für mindestens einen des einen oder der mehreren Benutzer einer BEM oberhalb eines Schwellenwerts zugeordnet ist; und
Neukonfigurieren der Anwendung mit dem neuen Anwendungsmerkmal basierend auf mindestens einem von der einen oder mehreren BEMs und der programmatischen Ergebniskennzahl.

2. System nach Anspruch 1, wobei die Konfiguration des Prozessors zur Identifizierung des Ziels umfasst, dass der Prozessor konfiguriert ist zum:

Empfangen von Benutzereingaben bezüglich dessen, was der Nutzer in Bezug auf seine Diabeteserkrankung erreichen möchte; und
Umwandeln der Benutzereingabe in das Ziel basierend auf einer oder mehrerer definierter Richtlinien, und optional, wobei die Konfiguration des Prozessors zur Umwandlung der Benutzereingabe in das Ziel basierend auf der einen oder den mehreren definierten Richtlinien umfasst, dass der Prozessor konfiguriert ist zum:
Kategorisieren des Benutzers in eine Kategorie basierend auf den Richtlinien und den dem Benutzer zugeordneten Informationen, wobei:

die dem Benutzer zugeordneten Informationen die Klassifizierungsinformationen einschließen und die Richtlinien das Ziel für die Kategorie angeben; und
Auswählen des Ziels für den Benutzer basierend auf der Kategorisierung.

3. System nach Anspruch 1, wobei die Konfiguration des Prozessors zur Identifizierung des Ziels umfasst, dass der Prozessor konfiguriert ist zum:

Empfangen von Benutzereingaben bezüglich dessen, was der Nutzer in Bezug auf seine Diabeteserkrankung erreichen möchte; und
Umwandeln der Benutzereingaben in das Ziel basierend auf den Informationen, die der Benutzergruppe zugeordnet sind.

4. System nach Anspruch 1, wobei die Konfiguration des Prozessors zur Neukonfigurierung der Anwendung mit dem neuen Anwendungsmerkmal umfasst, dass der Prozessor konfiguriert ist zum:

Identifizieren eines leistungsschwachen Anwendungsmerkmals aus dem einen oder den mehreren Anwendungsmerkmalen mit einer entsprechenden BEM, die unterhalb eines Schwellenwerts liegt; und
Ersetzen des leistungsschwachen Anwendungsmerkmals des einen oder der mehreren Anwendungsmerkmale durch das neue Anwendungsmerkmal, und optional, wobei die Konfiguration des Prozessors zur Neukonfiguration der Anwendung mit dem neuen Anwendungsmerkmal umfasst, dass der Prozessor konfiguriert ist zum:

Identifizieren, dass das leistungsschwache Anwendungsmerkmal des einen oder der mehreren Anwendungsmerkmale sich auf das Ziel bezieht; und

Identifizieren, dass die programmatische Ergebniskennzahl unterhalb eines Schwellenwerts liegt.

5. Verfahren zum Konfigurieren einer Anwendung mit einer oder mehreren Anwendungsmerkmalen, umfassend:

Empfangen einer Anfrage zur Konfiguration der Anwendung für die Verwendung durch einen Benutzer, wobei die Anwendung mindestens teilweise auf einer Rechenvorrichtung vorhanden ist, um Sensordaten zu verwalten, die von einem dem Benutzer zugeordneten Glukoseüberwachungssystem erzeugt werden;

Identifizieren eines Ziels für den Benutzer;

Identifizieren von Klassifizierungsinformationen, die dem Benutzer zugeordnet sind, wobei die Klassifizierungsinformationen mindestens eine der folgenden Informationen einschließen: Ziel, Interessen, Fähigkeiten, demografische Informationen, Krankheitsverlauf oder Medikamentenplan des Benutzers;

Auswählen einer Benutzergruppe aus einem Benutzerpool basierend auf einer oder mehreren Ähnlichkeiten zwischen dem Benutzer und der Benutzergruppe in Bezug auf die identifizierten Klassifizierungsinformationen;

Identifizieren des einen oder der mehreren Anwendungsmerkmale aus einer Vielzahl von Anwendungsmerkmalen basierend auf dem Ziel des Benutzers und einer Korrelation jedes der Vielzahl von Anwendungsmerkmalen mit dem Ziel in einem der Benutzergruppe zugeordneten Datensatz; und

Konfigurieren der Anwendung mit dem einen oder den mehreren Anwendungsmerkmalen;

Empfangen einer Vielzahl von Eingaben, die Folgendes einschließen:

eine erste Eingabe, die vom Glukoseüberwachungssystem erzeugte und dem Benutzer zugeordnete Glukosemesswerte einschließt; und

eine zweite Eingabe, die das Verhalten des Benutzers in Bezug auf das eine oder die mehreren Anwendungsmerkmale anzeigt;

Berechnen einer programmatischen Ergebniskennzahl, die dem Ziel zugeordnet ist, basierend mindestens auf der ersten Eingabe, wobei die programmatische Ergebniskennzahl angibt, inwieweit der Benutzer das Ziel erreicht hat;

basierend auf der zweiten Eingabe, Berechnen einer oder mehrerer Verhaltensnutzungskennzahlen (BEM) für das eine oder die mehreren Anwendungsmerkmale, so dass für jedes des einen oder der mehreren Anwendungsmerkmale eine separate BEM berechnet wird;

Identifizieren eines oder mehrerer Benutzer in der ausgewählten Benutzergruppe oder dem Benutzerpool, deren BEMs der einen oder den mehreren berechneten BEMs ähnlich sind;

Identifizieren eines neuen Anwendungsmerkmals, das nicht in dem einen oder den mehreren Merkmalen enthalten ist, basierend darauf, dass das Merkmal für mindestens einen des einen oder der mehreren Benutzer einer BEM oberhalb eines Schwellenwerts zugeordnet ist; und

Neukonfigurieren der Anwendung mit dem neuen Anwendungsmerkmal basierend auf mindestens einem von der einen oder mehreren BEMs und der programmatischen Ergebniskennzahl.

6. Verfahren nach Anspruch 5, wobei die Identifizierung des Ziels ferner Folgendes umfasst:

Empfangen von Benutzereingaben bezüglich dessen, was der Nutzer in Bezug auf seine Diabeteserkrankung erreichen möchte; und

Umwandeln der Benutzereingabe in das Ziel basierend auf einer oder mehreren definierten Richtlinien, und optional, wobei das Umwandeln ferner Folgendes umfasst:

Kategorisieren des Benutzers in eine Kategorie basierend auf den Richtlinien und den dem Benutzer zugeordneten Informationen, wobei:

die dem Benutzer zugeordneten Informationen die Klassifizierungsinformationen einschließen und die Richtlinien das Ziel für die Kategorie angeben; und

Auswählen des Ziels für den Benutzer basierend auf der Kategorisierung.

7. Verfahren nach Anspruch 5, wobei die Identifizierung des Ziels Folgendes umfasst:

Empfangen von Benutzereingaben bezüglich dessen, was der Nutzer in Bezug auf seine Diabeteserkrankung erreichen möchte; und

Umwandeln der Benutzereingaben in das Ziel basierend auf den Informationen, die der Benutzergruppe zugeordnet sind.

8. System nach Anspruch 3 oder Verfahren nach Anspruch 7, wobei die der Benutzergruppe zugeordneten Informationen eine oder mehrere glukosebezogene Kennzahlen der Benutzergruppe einschließen.

9. System nach Anspruch 1 oder Verfahren nach Anspruch 5, wobei das Auswählen der Benutzergruppe ferner auf einer programmatischen Ergebniskennzahl jedes Benutzers aus dem Benutzerpool in Bezug auf das Ziel basiert, und optional, wobei:

   die programmatischen Ergebniskennzahlen der ausgewählten Benutzergruppe oberhalb eines Schwellenwerts für die programmatische Ergebniskennzahl liegen, welcher der Zielerreichung zugeordnet ist, und
   der Schwellenwert für die programmatische Ergebniskennzahl, die der Zielerreichung zugeordnet ist, einen definierten Mindestmenge an positivem fortschritt in Richtung Zielerreichung angibt.

10. System nach Anspruch 1 oder Verfahren nach Anspruch 5, wobei die Korrelation jedes der Vielzahl von Anwendungsmerkmalen mit dem Ziel eine Korrelation jedes der Vielzahl von Anwendungsmerkmalen mit der Zielerreichung umfasst, oder wobei die Korrelation jedes der Vielzahl von Anwendungsmerkmalen mit dem Ziel auf einer Anzahl von Benutzern in der ausgewählten Benutzergruppe basiert, die das Merkmal verwendet haben, und auf der Verhaltensnutzung der Anzahl von Benutzern in Bezug auf das Merkmal, und optional, wobei die Verhaltensnutzung jedes Benutzers der Anzahl von Benutzern in Bezug auf das Anwendungsmerkmal durch eine Verhaltensnutzungskennzahl (BEM) jedes Benutzers der Anzahl von Benutzern in Bezug auf das Anwendungsmerkmal angezeigt wird, und wobei die BEM auf einer Interaktion jedes Benutzers der Anzahl von Benutzern mit dem Merkmal basiert, wobei die Interaktion mindestens eines der Folgenden einschließt:

   eine Häufigkeit, mit der jeder Benutzer der Anzahl von Benutzern mit dem Anwendungsmerkmal interagiert;
   eine Häufigkeit, mit der jeder Benutzer der Anzahl von Benutzern eine von dem Anwendungsmerkmal generierte Empfehlung ignoriert;
   eine durchschnittliche Zeit, die jeder Benutzer der Anzahl von Benutzern mit der Interaktion mit dem Anwendungsmerkmal verbringt; oder
   wie genau das Verhalten jedes Benutzers der Anzahl von Benutzern der von dem Anwendungsmerkmal generierten Empfehlung entspricht.

11. System nach Anspruch 4 oder Verfahren nach Anspruch 5, wobei jede BEM der einen oder mehreren BEMs auf einer Interaktion des Benutzers mit einem entsprechenden Anwendungsmerkmal des einen oder der mehreren Anwendungsmerkmale basiert, wobei die Interaktion mindestens eines der Folgenden einschließt:

   eine Häufigkeit, mit der der Benutzer mit dem entsprechenden Anwendungsmerkmal interagiert;
   eine Häufigkeit, mit der der Benutzer eine von dem entsprechenden Anwendungsmerkmal generierte Empfehlung ignoriert;
   eine durchschnittliche Zeit, die der Benutzer mit der Interaktion mit dem entsprechenden Anwendungsmerkmal verbringt; oder
   wie genau das Verhalten des Benutzers der von dem entsprechenden Anwendungsmerkmal generierten Empfehlung entspricht.

12. Verfahren nach Anspruch 11, wobei das Rekonfigurieren der Anwendung mit dem neuen Anwendungsmerkmal Folgendes umfasst:

   Identifizieren eines leistungsschwachen Anwendungsmerkmals aus dem einen oder den mehreren Anwendungsmerkmalen mit einer entsprechenden BEM, die unterhalb eines Schwellenwerts liegt; und
   Ersetzen des leistungsschwachen Anwendungsmerkmals des einen oder der mehreren Anwendungsmerkmale durch das neue Anwendungsmerkmal, und optional, wobei das Rekonfigurieren der Anwendung mit dem neuen Anwendungsmerkmal Folgendes umfasst:

      Identifizieren, dass das leistungsschwache Anwendungsmerkmal des einen oder der mehreren Anwendungsmerkmale sich auf das Ziel bezieht; und
      Identifizieren, dass die programmatische Ergebniskennzahl unterhalb eines Schwellenwerts liegt.

13. System nach Anspruch 1 oder Verfahren nach Anspruch 5, wobei jedes des einen oder der mehreren Anwendungsmerkmale eine Korrelation mit dem Ziel aufweist, die oberhalb eines Korrelationsschwellenwerts liegt, oder wobei jedes des einen oder der mehreren Anwendungsmerkmale eine Merkmalseinstellung umfasst.

**14.** Nichtflüchtiges, computerlesbares Medium, auf dem Anweisungen gespeichert sind, die, wenn sie von einem Prozessor ausgeführt werden, ein Computersystem veranlassen, das Verfahren nach einem der Ansprüche 5 bis 13 durchzuführen.

**Revendications**

**1.** Système comprenant :

un circuit de mémoire ; et
un processeur configuré pour :

recevoir une demande de configuration d'une application destinée à être utilisée par un utilisateur, dans lequel l'application est au moins partiellement installée sur un dispositif informatique pour gérer des données de capteur générées par un système de surveillance de glucose associé à l'utilisateur ;
identifier un objectif pour l'utilisateur ;
identifier des informations de classification associées à l'utilisateur, les informations de classification incluant au moins une parmi des informations d'objectif, d'intérêt, de capacité, démographiques, informations sur l'évolution de la maladie ou informations sur le régime médicamenteux de l'utilisateur ;
sélectionner un groupe d'utilisateurs parmi un bassin d'utilisateurs sur la base d'une ou de plusieurs similarités entre l'utilisateur et le groupe d'utilisateurs par rapport aux informations de classification identifiées ;
identifier une ou plusieurs fonctionnalités d'application parmi une pluralité de fonctionnalités d'application sur la base de l'objectif de l'utilisateur et d'une corrélation de chacune de la pluralité de fonctionnalités d'application et l'objectif dans un ensemble de données associé au groupe d'utilisateurs ; et

configurer automatiquement l'application avec l'une ou les plusieurs fonctionnalités d'application, dans lequel le processeur est en outre configuré pour :
recevoir une pluralité d'entrées incluant :

une première entrée incluant des mesures du glucose associées à l'utilisateur générées par le système de surveillance du glucose ; et
une seconde entrée indicative du comportement de l'utilisateur par rapport à l'une ou aux plusieurs fonctionnalités d'application ;
calculer une métrique de résultat programmatique associée à l'objectif sur la base d'au moins la première entrée, dans lequel la métrique de résultat programmatique est indicative d'une mesure dans laquelle l'utilisateur a accompli l'objectif ;
calculer, sur la base de la seconde entrée, une ou plusieurs métriques d'engagement comportemental (BEM) pour l'une ou les plusieurs fonctionnalités d'application, de sorte qu'une BEM séparée est calculée pour chacune de l'une ou des plusieurs fonctionnalités d'application ;
identifier un ou plusieurs utilisateurs dans le groupe d'utilisateurs ou pool d'utilisateurs sélectionné avec des BEM similaires à l'une ou aux plusieurs BEM calculés ;
identifier une nouvelle fonctionnalité d'application non incluse dans l'une ou les plusieurs fonctionnalités sur la base de la fonctionnalité étant associée à une BEM supérieure à un seuil pour au moins un parmi l'un ou les plusieurs utilisateurs ; et
reconfigurer l'application avec la nouvelle fonctionnalité d'application sur la base de l'au moins une parmi l'une ou les plusieurs BEM et de la métrique de résultat programmatique.

**2.** Système selon la revendication 1, dans lequel le processeur étant configuré pour identifier l'objectif comprend le processeur étant configuré pour :

recevoir une entrée utilisateur concernant ce que l'utilisateur prévoit d'accomplir par rapport au diabète de l'utilisateur ; et
convertir l'entrée utilisateur en l'objectif sur la base d'une ou de plusieurs lignes directrices définies, et facultativement, dans lequel le processeur étant configuré pour convertir l'entrée utilisateur en l'objectif sur la base de l'une ou des plusieurs lignes directrices définies comprend le processeur étant configuré pour :
catégoriser l'utilisateur dans une catégorie sur la base des lignes directrices et d'informations associées à l'utilisateur, dans lequel :

les informations associées à l'utilisateur incluent les informations de classification, et les lignes directrices indiquent l'objectif pour la catégorie ; et

sélectionner l'objectif pour l'utilisateur sur la base de la catégorisation.

3. Système selon la revendication 1, dans lequel le processeur étant configuré pour identifier l'objectif comprend le processeur étant configuré pour :

recevoir une entrée utilisateur concernant ce que l'utilisateur prévoit d'accomplir par rapport au diabète de l'utilisateur ; et

convertir l'entrée utilisateur en l'objectif sur la base d'informations associées au groupe d'utilisateurs.

4. Système selon la revendication 1, dans lequel le processeur étant configuré pour reconfigurer l'application avec la nouvelle fonctionnalité d'application comprend le processeur étant configuré pour :

identifier une fonctionnalité d'application à faible performance parmi l'une ou les plusieurs fonctionnalités d'application avec une BEM correspondante qui est inférieure à un seuil ; et

remplacer la fonctionnalité d'application à faible performance de l'une ou des plusieurs fonctionnalités d'application par la nouvelle fonctionnalité d'application, et facultativement, dans lequel le processeur étant configuré pour reconfigurer l'application avec la nouvelle fonctionnalité d'application comprend le processeur étant configuré pour :

identifier que la fonctionnalité d'application à faible performance parmi l'une ou les plusieurs fonctionnalités d'application concerne l'objectif ; et

identifier que la métrique de résultat programmatique est inférieure à un seuil.

5. Procédé de configuration d'une application avec une ou plusieurs fonctionnalités d'application, comprenant :

la réception d'une demande de configuration de l'application destinée à être utilisée par un utilisateur, dans lequel l'application est au moins partiellement installée sur un dispositif informatique pour gérer des données de capteur générées par un système de surveillance de glucose associé à l'utilisateur ;

l'identification d'un objectif pour l'utilisateur ;

l'identification d'informations de classification associées à l'utilisateur, les informations de classification incluant au moins une parmi des informations d'objectif, d'intérêt, de capacité, démographiques, informations sur l'évolution de la maladie ou informations sur le régime médicamenteux de l'utilisateur ;

la sélection d'un groupe d'utilisateurs parmi un bassin d'utilisateurs sur la base d'une ou de plusieurs similarités entre l'utilisateur et le groupe d'utilisateurs par rapport aux informations de classification identifiées ; l'identification d'une ou de plusieurs fonctionnalités d'application parmi une pluralité de fonctionnalités d'application sur la base de l'objectif de l'utilisateur et d'une corrélation de chacune de la pluralité de fonctionnalités d'application et l'objectif dans un ensemble de données associé au groupe d'utilisateurs ;

la configuration de l'application avec l'une ou les plusieurs fonctionnalités d'application ;

la réception d'une pluralité d'entrées incluant :

une première entrée incluant des mesures du glucose associées à l'utilisateur générées par le système de surveillance du glucose ; et

une deuxième entrée indicative du comportement de l'utilisateur par rapport à une ou plusieurs fonctionnalités de l'application ;

le calcul d'une métrique de résultat programmatique associée à l'objectif sur la base d'au moins la première entrée, dans lequel la métrique de résultat programmatique est indicative d'une mesure dans laquelle l'utilisateur a accompli l'objectif ;

le calcul, sur la base de la seconde entrée, d'une ou de plusieurs métriques d'engagement comportemental (BEM) pour l'une ou les plusieurs fonctionnalités d'application, de sorte qu'une BEM séparée est calculée pour chacune de l'une ou des plusieurs fonctionnalités d'application ;

l'identification d'un ou de plusieurs utilisateurs dans le groupe d'utilisateurs ou pool d'utilisateurs sélectionné avec des BEM similaires à l'une ou aux plusieurs BEM calculés ;

l'identification d'une nouvelle fonctionnalité d'application non incluse dans l'une ou les plusieurs fonctionnalités sur la base de la fonctionnalité étant associée à une BEM supérieure à un seuil pour au moins un parmi l'un ou les plusieurs utilisateurs ; et

la reconfiguration de l'application avec la nouvelle fonctionnalité d'application sur la base de l'au moins une parmi l'une ou les plusieurs BEM et de la métrique de résultat programmatique.

6. Procédé selon la revendication 5, dans lequel l'identification de l'objectif comprend en outre :

la réception d'une entrée utilisateur concernant ce que l'utilisateur prévoit d'accomplir par rapport au diabète de l'utilisateur ; et
la conversion de l'entrée utilisateur en l'objectif sur la base d'une ou de plusieurs lignes directrices définies, et facultativement, dans lequel la conversion comprend en outre :
la catégorisation de l'utilisateur dans une catégorie sur la base des lignes directrices et d'informations associées à l'utilisateur, dans lequel :

les informations associées à l'utilisateur incluent les informations de classification, et les lignes directrices indiquent l'objectif pour la catégorie ; et
la sélection de l'objectif pour l'utilisateur sur la base de la catégorisation.

7. Procédé selon la revendication 5, dans lequel l'identification de l'objectif comprend :

la réception d'une entrée utilisateur concernant ce que l'utilisateur prévoit d'accomplir par rapport au diabète de l'utilisateur ; et
la conversion de l'entrée utilisateur en l'objectif sur la base d'informations associées au groupe d'utilisateurs.

8. Système selon la revendication 3 ou procédé selon la revendication 7, dans lequel les informations associées au groupe d'utilisateurs incluent une ou plusieurs métriques liées au glucose du groupe d'utilisateurs.

9. Système selon la revendication 1 ou procédé selon la revendication 5, dans lequel la sélection du groupe d'utilisateurs est en outre basée sur une métrique de résultat programmatique de chacun parmi le bassin d'utilisateurs par rapport à l'objectif, et facultativement, dans lequel :

des métriques de résultat programmatique du groupe d'utilisateurs sélectionné sont supérieurs à un seuil de métrique de résultat programmatique associé à l'accomplissement de l'objectif, et
la métrique de résultat programmatique de seuil associé à l'accomplissement de l'objectif est indicative d'une quantité minimale définie de progrès positif vers l'accomplissement de l'objectif.

10. Système selon la revendication 1 ou procédé selon la revendication 5, dans lequel la corrélation de chacune de la pluralité de fonctionnalités d'application avec l'objectif comprend une corrélation de chacune de la pluralité de fonctionnalités d'application avec l'accomplissement de l'objectif, ou, dans lequel la corrélation de chacune de la pluralité de fonctionnalités d'application avec l'objectif est basée sur un nombre d'utilisateurs dans le groupe d'utilisateurs sélectionné qui ont utilisé la fonctionnalité et l'engagement comportemental du nombre d'utilisateurs par rapport à la fonctionnalité, et facultativement, dans lequel l'engagement comportemental de chacun parmi le nombre d'utilisateurs par rapport à la fonctionnalité d'application est indiqué par une métrique d'engagement comportemental (BEM) pour chaque utilisateur par rapport à la fonctionnalité d'application, et dans lequel la BEM est basée sur une interaction de chaque utilisateur parmi le nombre d'utilisateurs avec la fonctionnalité, l'interaction incluant au moins un parmi :

une fréquence à laquelle chaque utilisateur du nombre d'utilisateurs interagit avec la fonctionnalité d'application ;
une fréquence à laquelle chaque utilisateur parmi le nombre d'utilisateurs ignore des recommandations générées par la fonctionnalité d'application ;
une quantité moyenne de temps que chaque utilisateur parmi le nombre d'utilisateurs passe à interagir avec la fonctionnalité d'application ; ou
dans quelle mesure le comportement de chaque utilisateur parmi le nombre d'utilisateurs adhère aux recommandations générées par la fonctionnalité d'application.

11. Système selon la revendication 4 ou procédé selon la revendication 5, dans lequel chaque BEM parmi l'une ou les plusieurs BEM est basé sur une interaction de l'utilisateur avec une fonctionnalité d'application correspondante parmi l'une ou les plusieurs fonctionnalités d'application, l'interaction incluant au moins un parmi :

une fréquence à laquelle l'utilisateur interagit avec la fonctionnalité d'application correspondante ;
une fréquence à laquelle l'utilisateur ignore des recommandations générées par la fonctionnalité d'application correspondante ;

une quantité moyenne de temps que l'utilisateur passe à interagir avec la fonctionnalité d'application correspondante ; ou

dans quelle mesure le comportement de l'utilisateur adhère aux recommandations générées par la fonctionnalité d'application correspondante.

12. Procédé selon la revendication 11, dans lequel la reconfiguration de l'application avec la nouvelle fonctionnalité d'application comprend :

l'identification d'une fonctionnalité d'application à faible performance parmi l'une ou les plusieurs fonctionnalités d'application avec une BEM correspondante qui est inférieure à un seuil ; et

le remplacement de la fonctionnalité d'application à faible performance parmi l'une ou les plusieurs fonctionnalités d'application par la nouvelle fonctionnalité d'application, et facultativement, dans lequel la reconfiguration de l'application avec la nouvelle fonctionnalité d'application comprend :

l'identification que la fonctionnalité d'application à faible performance parmi l'une ou les plusieurs fonctionnalité d'application concerne l'objectif ; et

l'identification que la métrique de résultat programmatique est inférieure à un seuil.

13. Système selon la revendication 1 ou procédé selon la revendication 5, dans lequel chacune parmi l'une ou les plusieurs fonctionnalités d'application présente une corrélation avec l'objectif qui est supérieure à un seuil de corrélation, ou, dans lequel chaque fonctionnalité d'application parmi l'une ou les plusieurs fonctionnalités d'application comprend un réglage de fonctionnalité.

14. Support lisible par ordinateur non transitoire sur lequel sont stockées des instructions qui, lorsqu'elles sont exécutées par un processeur, amènent un système informatique à réaliser le procédé selon l'une quelconque des revendications 5 à 13.

Fig. 1A

**Fig. 1B**

EP 4 143 751 B1

**INPUTS 210**

- Food consumption
- Activity
- Patient stats
- Insulin
- Sensors
- Blood glucose
- Time
- User input

**INPUTS 220**

- User input
- Calendar
- Activity
- Location
- Time
- Sensors
- Food consumption

106

Data Analysis Module 113

**OUTCOME METRICS 130**

- Metabolic rate
- Activity level
- Insulin sensitivity
- Insulin on board
- Meal state
- Health/Sickness
- Glucose level
- Glucose trend
- Disease stage
- Clinical
- Programmatic

**BEHAVIORAL METRICS 128**

- Behavioral engag. (BEM)
- Meal habits
- Disease treatment adherence
- Medication adherence
- Healthcare utilization
- Exercise regimen
- Behavioral state

**Fig. 2**

300

302

IDENTIFY OBJECTIVES OF A USER OF AN APPLICATION

304

IDENTIFY A STRATIFIED GROUP OF SIMILAR USERS FROM A USER DATABASE BASED ON INFORMATION ASSOCIATED WITH THE USER

306

CONFIGURE THE APPLICATION BASED ON INFORMATION ASSOCIATED WITH THE USER,

INCLUDING THE USER'S OBJECTIVES, AND FURTHER BASED ON INFORMATION RELATING TO THE STRATIFIED GROUP

308

DETERMINE ONE OR MORE BEHAVIORAL METRICS AND/OR OUTCOME METRICS OF THE USER

310

CONTINOUSLY ADAPT THE APPLICATION CONFIGURATION BASED ON INFORMATION RELATING TO AT LEAST SOME OF BEHAVIORAL AND/OR OUTCOME METRICS OF THE USERS AS WELL AS INFORMATION RELATING TO AT LEASE TOME OF BEHAVIORAL AND/OR OUTCOME METRICS OF THE STRATIFIED GROUP OF USERS

Fig. 3

Fig. 4A

EP 4 143 751 B1

|  | Obj. | Demo. Info | Disease Pro. | Med. Info | Interest | Ability | FFSC 1 | FFSC 2 | FFSC 3 | ... | FFSC N |
|---|---|---|---|---|---|---|---|---|---|---|---|
| User 1 | 1 | 77 | Type I | Insulin | Running | Time Lim. | 90% |  | 89% | ... | 30% |
| User 2 | 0 | 22 | Type II | Oral Med. | ... | ... | 30% | 80% | 10% | ... | 20% |
| User 3 |  | 44 | Type II | Oral Med. | ... | ... | 40% |  | 32% | ... | 20% |
| User 4 | 1 | 67 | Type I | Insulin | Swimming | Time Lim. | 92% | 20% | 79% | ... | 25% |
| ... | ... | ... | ... | ... | ... | ... | ... | ... | ... | ... | ... |
| User N | ... | ... | ... | ... | ... | ... | ... | ... | ... | ... | ... |

440

|  | Obj. | Demo. Info | Disease Pro. | Med. Info | Interest | Ability | FFSC 1 | FFSC 2 | FFSC 3 | ... | FFSC N |
|---|---|---|---|---|---|---|---|---|---|---|---|
| User 1 | 1 | 77 | Type I | Insulin | Running | Time Lim. | 90% |  | 89% | ... | 30% |
| User 4 | 1 | 67 | Type I | Insulin | Swimming | Time Lim. | 92% | 20% | 79% | ... | 25% |
| User 8 | 1 | 72 | Type I | Insulin | Swimming | Time Lim. | 30% | 89% | 23% | ... | 90% |
| ... | ... | ... | ... | ... | ... | ... | ... | ... | ... | ... | ... |

441

442

|  | FFSC 1 | FFSC 2 | FFSC 3 | ... | FFSC N |
|---|---|---|---|---|---|
| User 102 | 93% | 18% |  | ... | 25% |
| User 1 | 90% |  | 89% | ... | 30% |
| User 4 | 92% | 20% | 79% | ... | 25% |
| User 8 | 30% | 89% | 23% | ... | 90% |
| ... | ... | ... | ... | ... | ... |

490

441

Fig. 4B

EP 4 143 751 B1

## 302

**User profile 116**

Demographic Info 118

Disease Progression Info 120

Medication Info 122

**Application Data 126**

Objectives 132

Interests 134

Abilities 136

## 304

User Group 1

User Group 2

User Group 3

...

User Group 4

## 306, 308, and 310

Training Data Set 540

Machine Learning Model 550

Output 548

App. Configuration 108, 508

Output 560

Model 451

Dataset 442

Behavioral metrics 128
Outcome metrics 130

Fig. 5A

EP 4 143 751 B1

540

| | Interest 1 | ... | Interest N | Ability 1 | ... | Ability N | FFSC 1 | FFSC 2 | FFSC 3 | ... | FFSC N |
|---|---|---|---|---|---|---|---|---|---|---|---|
| User 1 | 1 | ... | 1 | 1 | ... | 1 | 90% | 79% | 89% | ... | 30% |
| User 2 | 0 | ... | 0 | 0 | ... | 0 | 92% | 20% | 79% | ... | 25% |
| User 3 | 1 | ... | 0 | 1 | ... | 1 | 30% | 89% | 23% | ... | 90% |
| User 4 | 1 | ... | 1 | 0 | ... | 0 | ... | ... | ... | ... | ... |
| ... | ... | ... | ... | ... | ... | ... | ... | ... | ... | ... | ... |
| User N | ... | ... | ... | ... | ... | ... | ... | ... | ... | ... | ... |

X ⟵⟶ Y

542

| | Interest | ... | Ability 1 | ... | Ability N |
|---|---|---|---|---|---|
| User 1 | 1 | ... | 1 | ... | 1 |

544

| FFSC | FFSC 1 | FFSC 2 | FFSC 3 | FFSC 4 | ... | FFSC N |
|---|---|---|---|---|---|---|
| PBEMs | 90% | 92% | 50% | 20% | ... | ... |

546

| | Interest | ... | Ability 1 | ... | Ability N |
|---|---|---|---|---|---|
| User 102 | 0 | ... | 1 | ... | 0 |

548

| FFSC | FFSC 1 | FFSC 2 | FFSC 3 | FFSC 4 | ... | FFSC N |
|---|---|---|---|---|---|---|
| PBEMs | 30% | 95% | 50% | 89% | ... | ... |

Fig. 5B

45

EP 4 143 751 B1

User Database 110

To/From Data
Communications Network

I/O Devices
635

I/O Interface
620

Processor
605

Network Interface
625

Interconnect (Bus)
630

Memory
610

AACM 115

DAM 113

Decision Support Engine 112

Storage
615

User Profile 116

Computing Device 600

Fig. 6

46

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63016784
- US 2018277246 A
- US 6001067 A
- US 20110027127 A1
- US 20060020187 A1
- US 20090137887 A1
- US 20070027385 A1
- US 7310544 B
- US 6931327 B
- US 20050043598 A
- US 20070032706 A
- US 20070016381 A
- US 20080033254 A
- US 20050203360 A
- US 20050154271 A
- US 20050192557 A
- US 20060222566 A
- US 20070203966 A
- US 20070208245 A
- US 20190336053 A
- US 827577